# EUROPÄISCHE PATENTSCHRIFT

(11) **EP 0 772 587 B1**
(45) Veröffentlichungstag und Bekanntmachung des Hinweises auf die Patenterteilung: **06.10.1999**
(21) Anmeldenummer: 95926911.9
(22) Anmeldetag: 17.07.1995
(51) Int. Cl.: C07C 257/12, C07C 259/14, A01N 37/52

(54) **N-ALKOXY-AMIDIN-DERIVATE UND DEREN VERWENDUNG ALS SCHÄDLINGSBEKÄMPFUNGSMITTEL**
N-ALKOXY-AMIDINE DERIVATIVES AND THEIR USE AS PESTICIDES
DERIVES DE N-ALCOXY-AMIDINE ET LEUR UTILISATION COMME PESTICIDES

(30) Priorität: 29.07.1994 DE 4426940
(43) Veröffentlichungstag der Anmeldung: 14.05.1997
(73) Patentinhaber: BAYER AG, 51368 Leverkusen (DE)
(72) Erfinder: HEINEMANN, Ulrich, D-42799 Leichlingen (DE); KRÜGER, Bernd-Wieland, D-51467 Bergisch Gladbach (DE); TIEMANN, Ralf, D-51375 Leverkusen (DE); DUTZMANN, Stefan, D-40721 Hilden (DE); STENZEL, Klaus, D-40595 Düsseldorf (DE)
(86) Internationale Anmeldenummer: EP9502797
(87) Internationale Veröffentlichungsnummer: WO9604239

(56) Entgegenhaltungen:
- EP-A- 0 398 692
- WO-A-92/13830

## Beschreibung

Die vorliegende Erfindung betrifft neue N-Alkoxy-amidin-Derivate, ein Verfahren zu ihrer Herstellung und ihre Verwendung als Schädlingsbekämpfungsmittel. Außerdem betrifft die Erfindung auch neue Zwischenprodukte und ein Verfahren zu deren Herstellung.

Es ist bekannt, daß verschiedene substituierte Alkoximino- und Alkoxymethylenacetamide fungizide Eigenschaften besitzen (vgl. z.B. EP-A 398 692, EP-A 468 775, DE-A 40 30 038 und WO-A 92/13 830).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue N-Alkoxy-amidin-Derivate der allgemeinen Formel (I) gefunden, 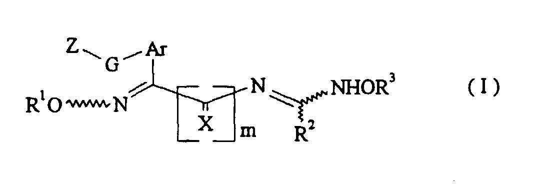 in welcher
- Ar: für jeweils gegebenenfalls substituiertes Arylen oder Heteroarylen steht;
- G: für eine Einfachbindung für Sauerstoff, für jeweils gegebenfalls durch Halogen, Hydroxy, Alkyl, Halogenalkyl oder Cycloalkyl substituiertes Alkandiyl, Alkendiyl, Oxaalkendiyl, Alkindiyl oder eine der nachstehenden Gruppierungen steht
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- oder -N=N-C(R⁴)=N-O-,
wobei
- n: für die Zahlen 0, 1 oder 2 steht,
- Q: für Sauerstoff oder Schwefel steht,
- R¹: für Wasserstoff oder Alkyl steht;
- R²: für Wasserstoff oder Alkyl steht;
- R³: für Wasserstoff oder Alkyl steht;
- R⁴: für Wasserstoff, Cyano oder jeweils gegebenenfalls substituiertes Alkyl, Alkoxy, Alkylthio, Alkylamino, Dialkylamino oder Cycloalkyl steht, und
- R⁵: für Wasserstoff, Hydroxy, Cyano oder jeweils gegebenenfalls substituiertes Alkyl, Alkoxy oder Cycloalkyl steht;
- X: für Sauerstoff oder Schwefel steht;
- m: für die Zahlen 0 oder 1 steht und
- Z: für jeweils gegebenenfalls substituiertes Alkyl, Alkenyl, Alkinyl, Cycloalkyl, Aryl oder Heterocyclyl steht.

Weiterhin wurde gefunden, daß man die neuen N-Alkoxy-amidin-Derivate der allgemeinen Formel (I) erhält, wenn man N-N-Dialkyl-amidin-Derivate der Formel (II) 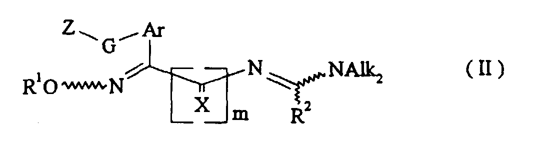 in welcher
- Ar, G, R¹, R², X, Z und m: die oben angegebene Bedeutung haben und
- Alk: für Alkyl steht,
mit Hydroxylamin-Derivaten der allgemeinen Formel (III)

H₂N―O―R³ (III)

in welcher
- R³: die oben angegebene Bedeutung hat,
oder mit deren Säureadditionssalzen in Gegenwart eines Verdünnungsmittels und gegebenenfalls in Gegenwart eines Reakionshilfsmittels umsetzt.

Schließlich wurde gefunden, daß die neuen N-Alkoxy-amidin-Derivate der allgemeinen Formel (I) starke fungizide Wirksamkeit zeigen.

Die erfindungsgemäßen Verbindungen können gegebenenfalls als Mischungen verschiedener möglicher isomerer Formen, insbesondere von E- und Z-Isomeren, vorliegen. Es werden sowohl die E- als auch die Z-Isomeren wie auch beliebige Mischungen dieser Isomeren beansprucht.

Gegenstand der Erfindung sind vorzugsweise Verbindungen der Formel (I), in welcher
- Ar: für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
- G: für eine Einfachbindung, für Sauerstoff, für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Oxaalkendiyl, Alkindiyl mit jeweils bis zu 4 Kohlenstoffatomen oder eine der nachstehenden Gruppierungen steht
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, N(R⁵)-CQ-Q-CH₂-, -CQ-CH₂- oder -N=N-C(R⁴)=N-=-,
wobei
- n: für die Zahlen 0, 1 oder 2 steht,
- Q: für Sauerstoff oder Schwefel steht,
- R⁴: für Wasserstoff, Cyano, für gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
- R⁵: für Wassersstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C1-C4-Alkoxy-carbonyl substituiertes Cycloalkyl mit 3 bis 6 Kohlenstoffatomen steht.
- R¹: für Wasserstoff oder geradkettiges oder verzweigtes Alkyl mit 1 bis 4 Kohlenstoffatomen steht.
- R²: für Wasserstoff oder geradkettiges oder verzweigtes Alkyl mit 1 bis 4 Kohlenstoffatomen steht.
- R³: für Wasserstoff oder geradkettiges oder verzweigtes Alkyl mit 1 bis 4 Kohlenstoffatomen steht.
- X: für Sauerstoff oder Schwefel steht.
- m: für die Zahlen 0 oder 1 steht und
- Z: für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxycarbonyl substiuiertes Cycloalkyl mit 3 bis 6 Kohlenstoffatomen, für jeweils gegebenenfalls substituiertes Phenyl, Naphthyl oder (gegebenenfalls benzannelliertes) Heterocyclyl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Sauerstoff (als Ersatz für zwei geminale Wasserstoffatome), Halogen, Cyano, Nitro, Amino, Hydroxy, Formyl, Carboxy, Carbamoyl Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen; jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylaminio, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen; Cyano, Nitro, Carboxy, Carbamoyl und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen und/oder Alkylcarbonyl oder Alkoxycarbonyl mit jeweils bis zu 5 Kohlenstoffatomen substituiertes Phenyl, Phenoxy, Benzyl, Benzyloxy, Phenylethyl oder Phenylethyloxy.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- Ar: für jeweils gegebenenfalls substituiertes ortho-, meta- oder para-Phenylen; für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl, Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio, Methylsulfinyl oder Methylsulfonyl.
- G: für eine Einfachbindung, für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- oder -N(R⁵)-CQ-Q-CH₂- steht,
wobei
- n: für die Zahlen 0, 1 oder 2 steht,
- Q: für Sauerstoff oder Schwefel steht,
- R⁴: für Wasserstoff, Cyano, für gegebenenfalls durch Fluor, Chlor, Cyano, Methoxy oder Ethoxy substituiertes Methyl, Ethyl, n- oder i-Propyl, n-, i-oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
- R⁵: für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht.
- R¹: für Wasserstoff, Methyl, Ethyl, n- oder i-Propyl sowie n-, i-, s- oder t-Butyl steht.
- R²: für Wasserstoff, Methyl, Ethyl, n- oder i-Propyl sowie n-, i-, s- oder t-Butyl steht.
- R³: für Wasserstoff, Methyl, Ethyl, n- oder i-Propyl sowie n-, i-, s- oder t-Butyl steht.
- X: für Sauerstoff oder Schwefel steht.
- m: für die Zahlen 0 oder 1 steht und
- Z: für gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl, für jeweils gegebenenfalls durch Fluor, Chlor, Brom, Cyano, Carboxy, Phenyl (welches gegebenenfalls durch Fluor, Chlor, Brom, Cyano, Methyl, Ethyl, n-oder i-Propyl, n-, i-, s- oder t-Butyl Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl, für jeweils gegebenenfalls substituiertes Phenyl, Naphthyl, Furyl, Tetrahydrofuryl, Benzofuryl, Tetrahydropyranyl, Thienyl, Benzothienyl, Pyrrolyl, Dihydropyrrolyl, Tetrahydropyrrolyl, Benzopyrrolyl, Benzodihydropyrrolyl, Oxazolyl, Benzoxazolyl, Isoxazolyl, Thiazolyl, Benzthiazolyl, Isothiazolyl, Imidazolyl, Benzimidazolyl, Oxadiazolyl, Thiadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Sauerstoff (als Ersatz für zwei geminale Wasserstoffatome), Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n-oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Nitro, Carboxy, Carbamoyl, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Acetyl, Methoxycarbonyl oder Ethoxycarbonyl substituiertes Phenyl, Phenoxy, Benzyl oder Benzyloxy.

Eine besonders bevorzugte Gruppe erfindungsgemäßer Verbindungen sind diejenigen Verbindungen der Formel (I), in welcher
- Ar: für ortho-Phenylen, Pyridin-2,3-diyl oder Thiophen-2,3-diyl steht,
- G: für Sauerstoff, Methylen oder eine der nachstehenden Gruppierungen
-CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -N(R⁵)- oder -CH₂-O-N=C(R⁴)- steht,
wobei
- n: für die Zahlen 0, 1 oder 2 steht,
- R⁴: für Wasserstoff, Methyl oder Ethyl steht und
- R⁵: für Wasserstoff, Methyl oder Ethyl steht.
- R¹: für Methyl steht.
- R²: für Wasserstoff oder Methyl steht.
- R³: für Wasserstoff oder Methyl steht.
- X: für Sauerstoff steht.
- m: für die Zahlen 0 oder 1 steht und
- Z: für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Beispiele für die erfindungsgemäßen Verbindungen sind in den Tabellen 1 bis 60 aufgeführt:

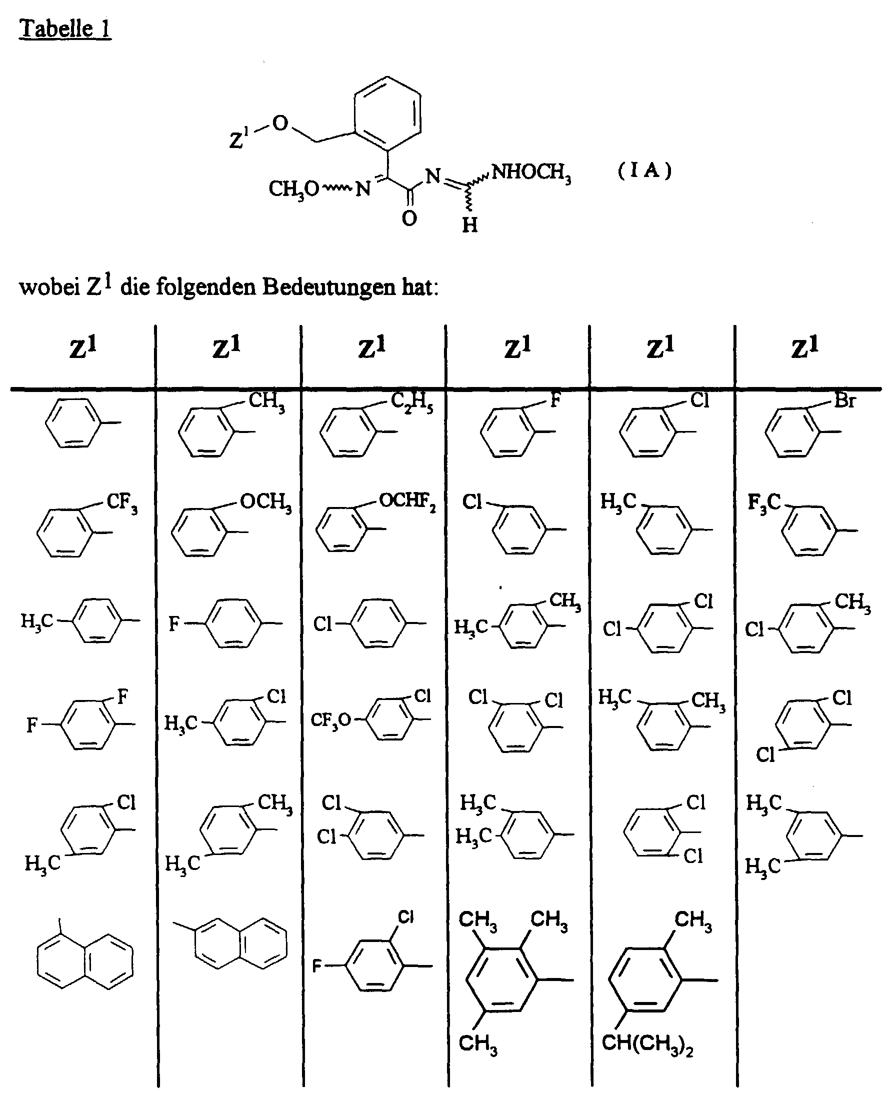

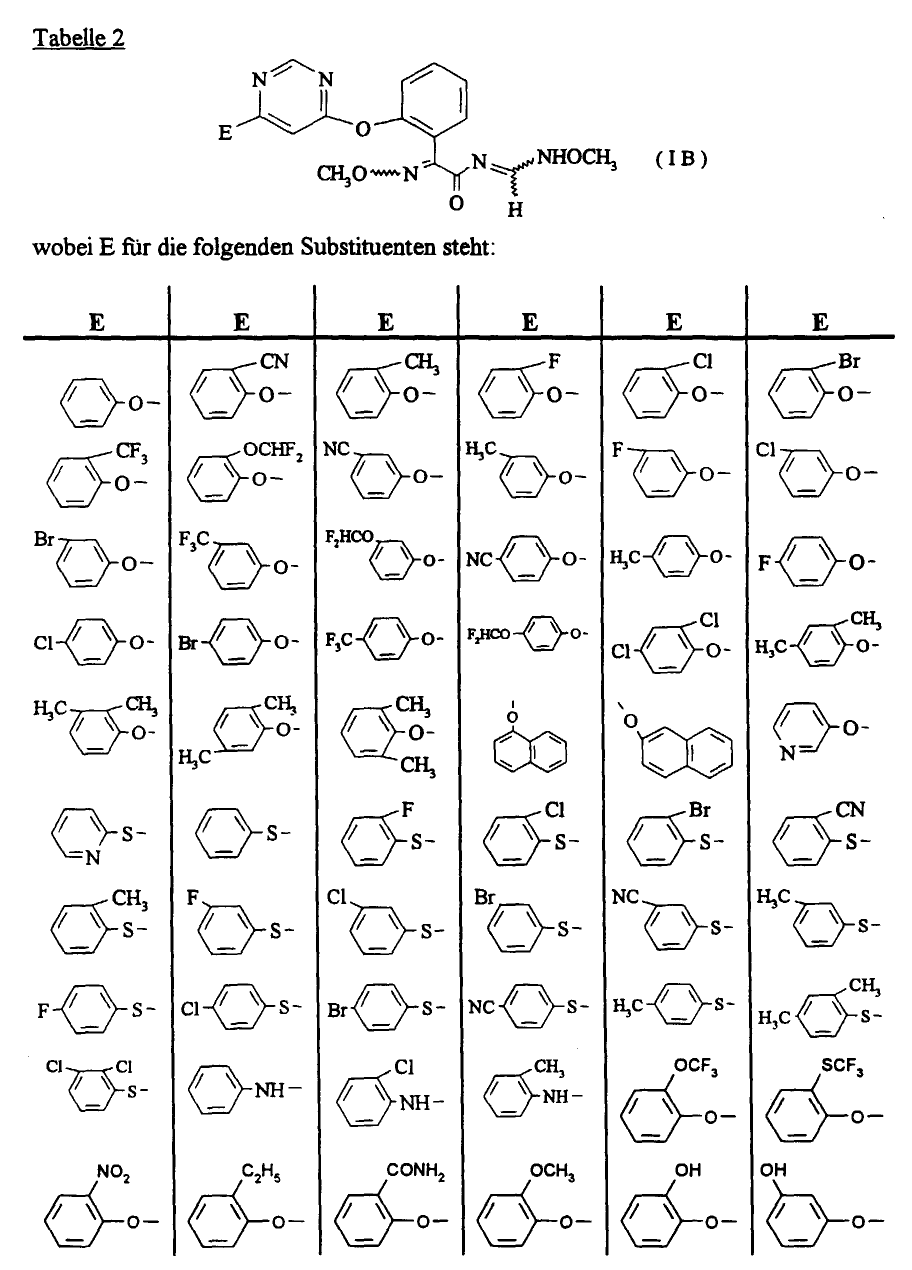

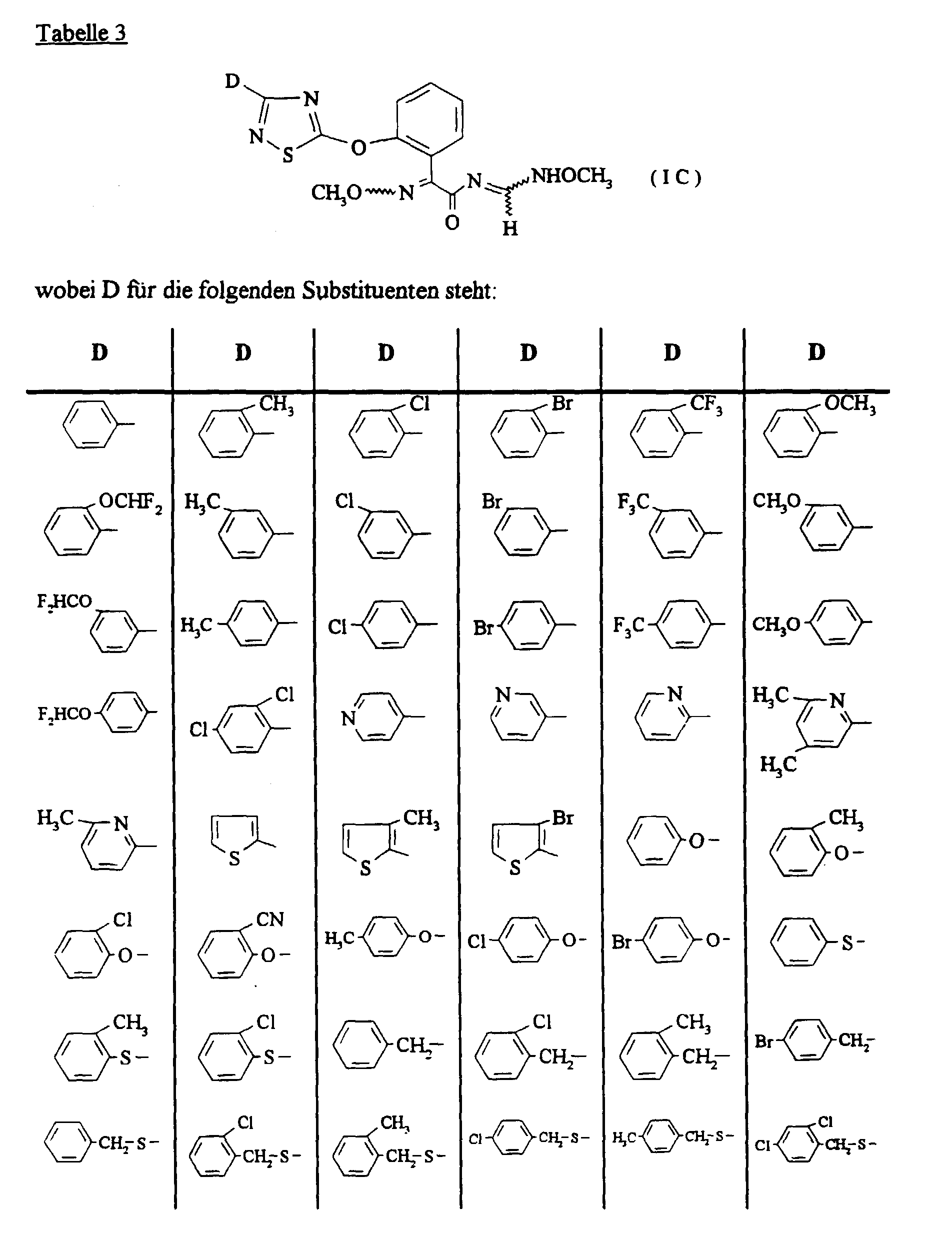

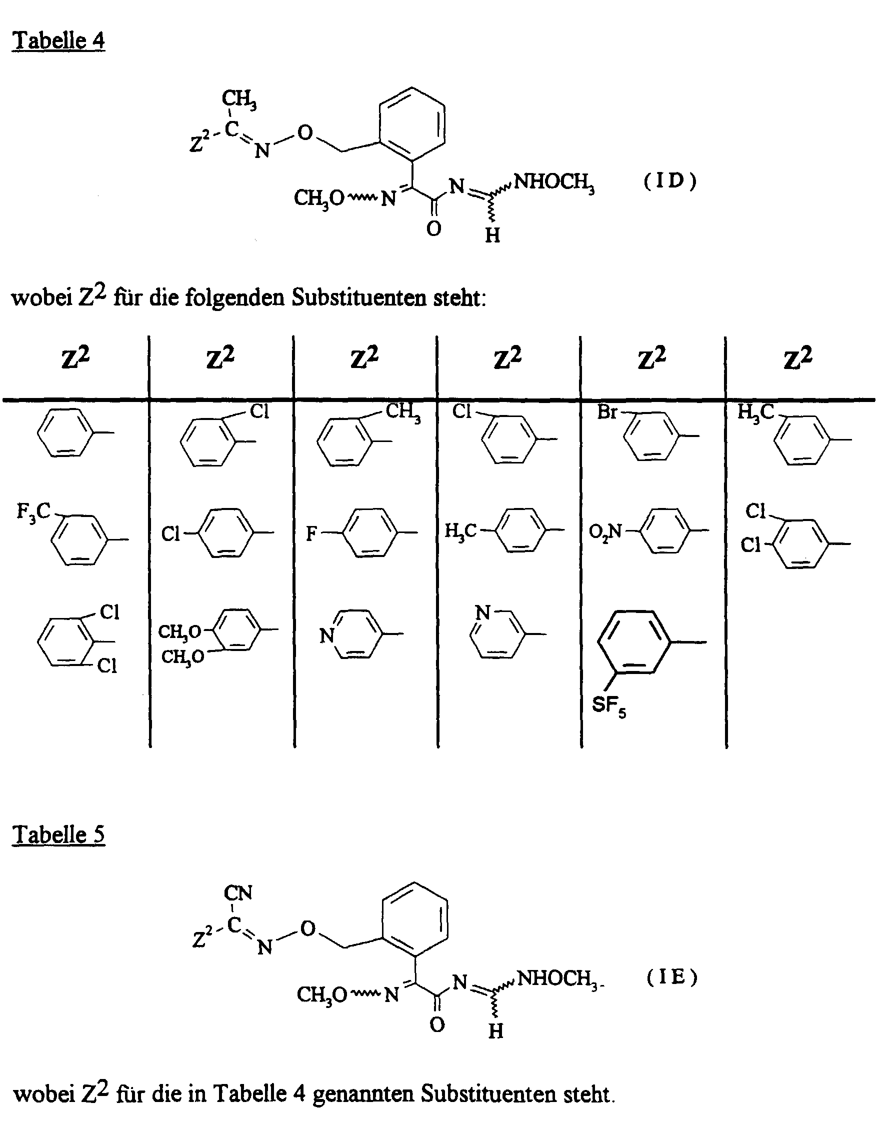

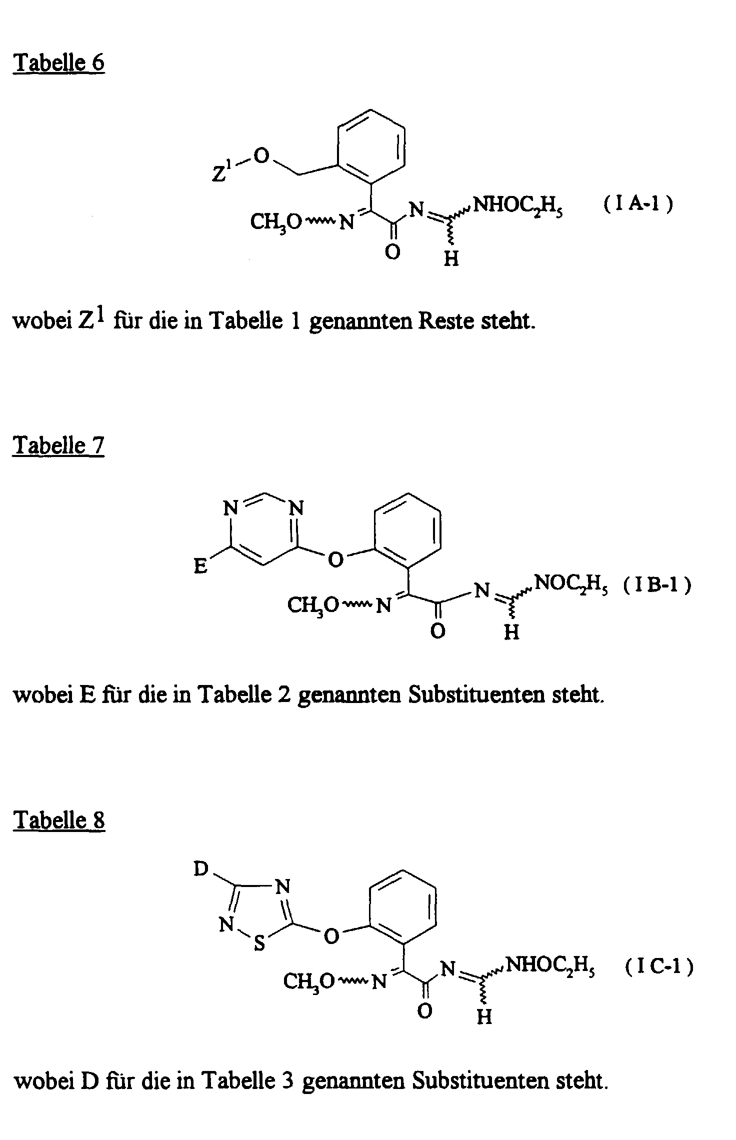

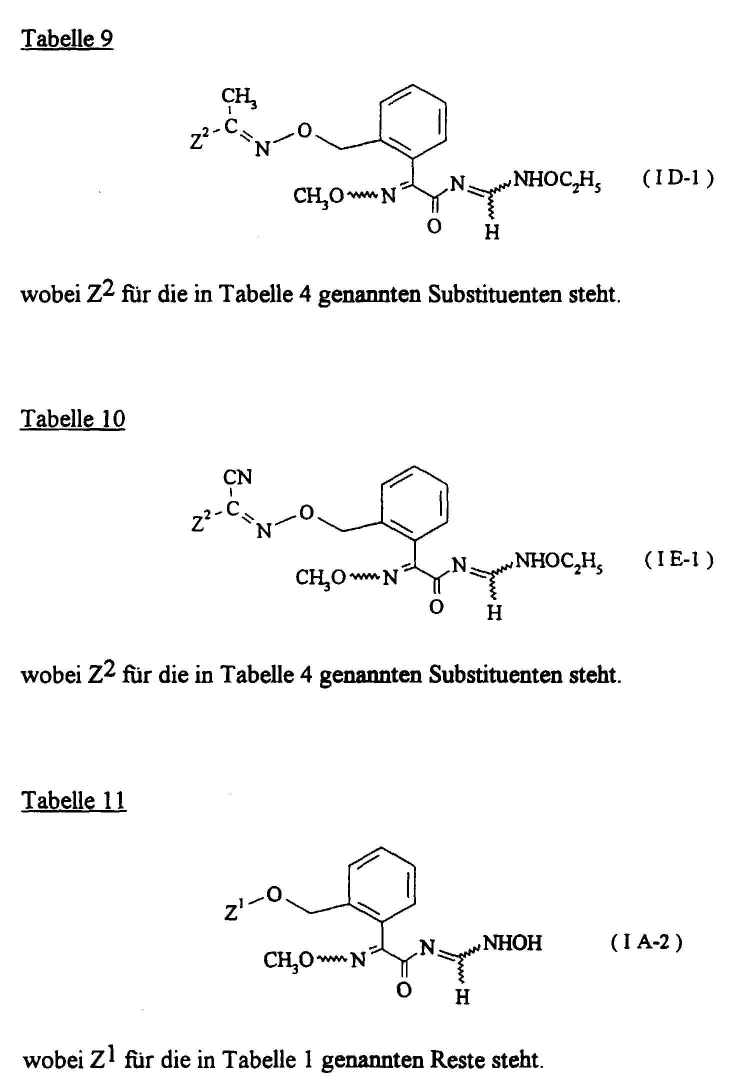

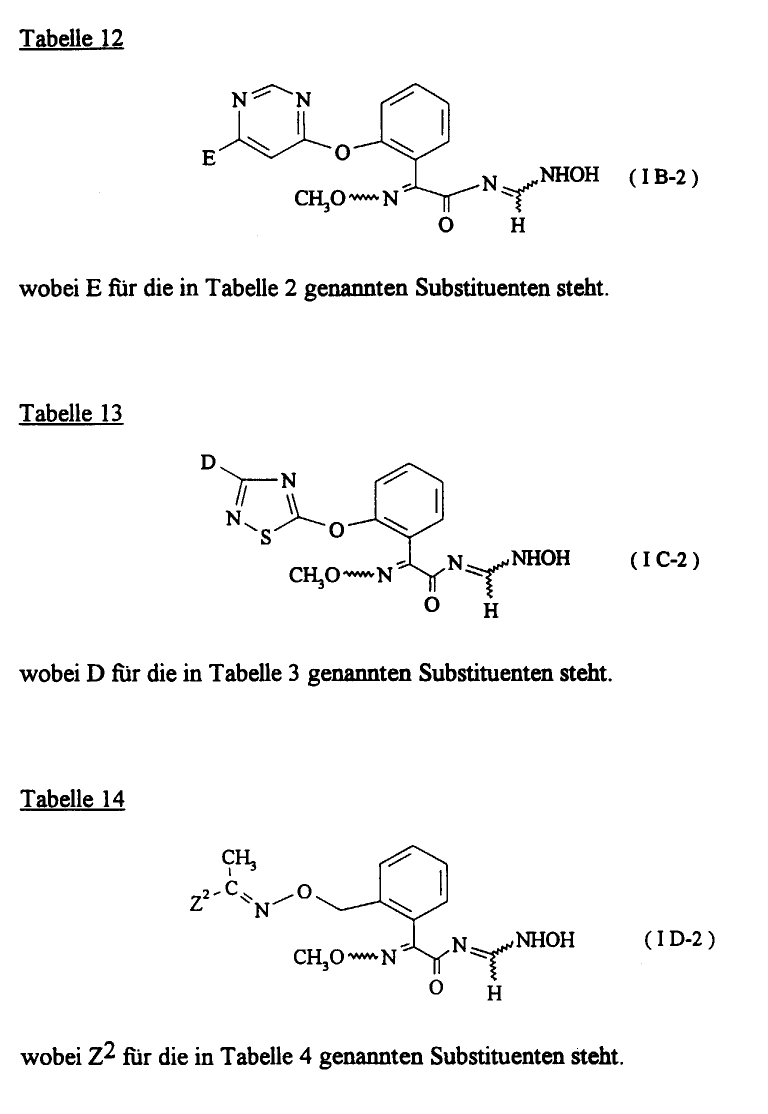

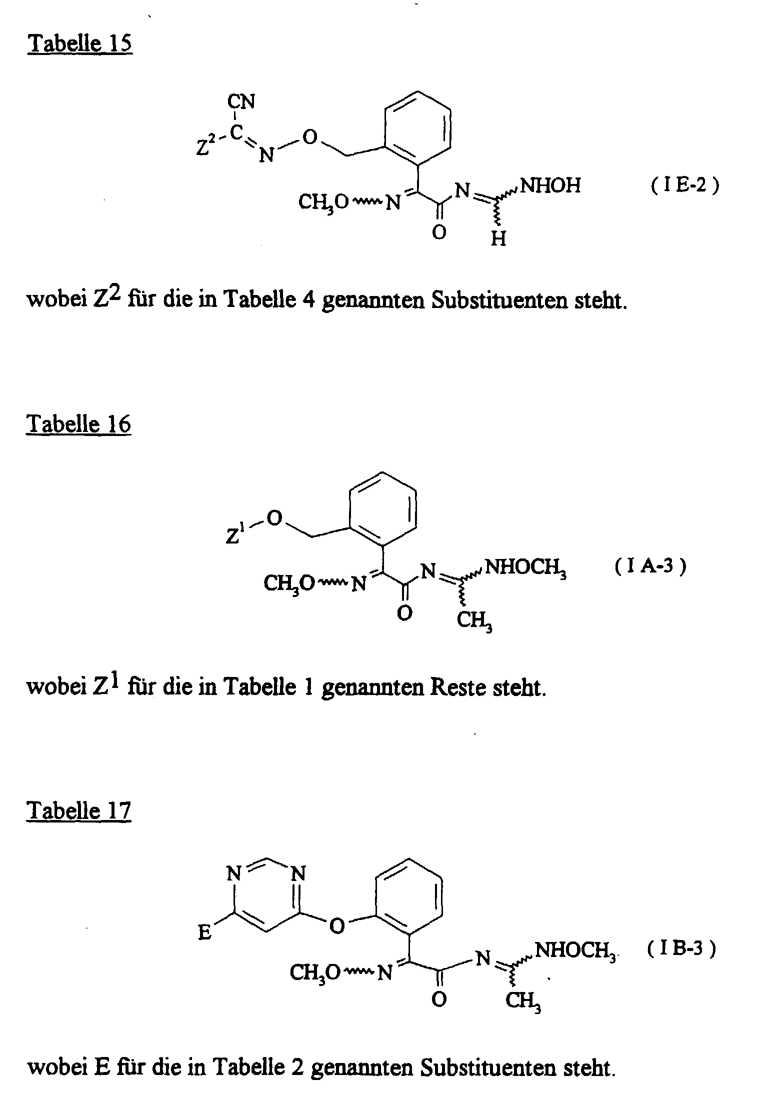

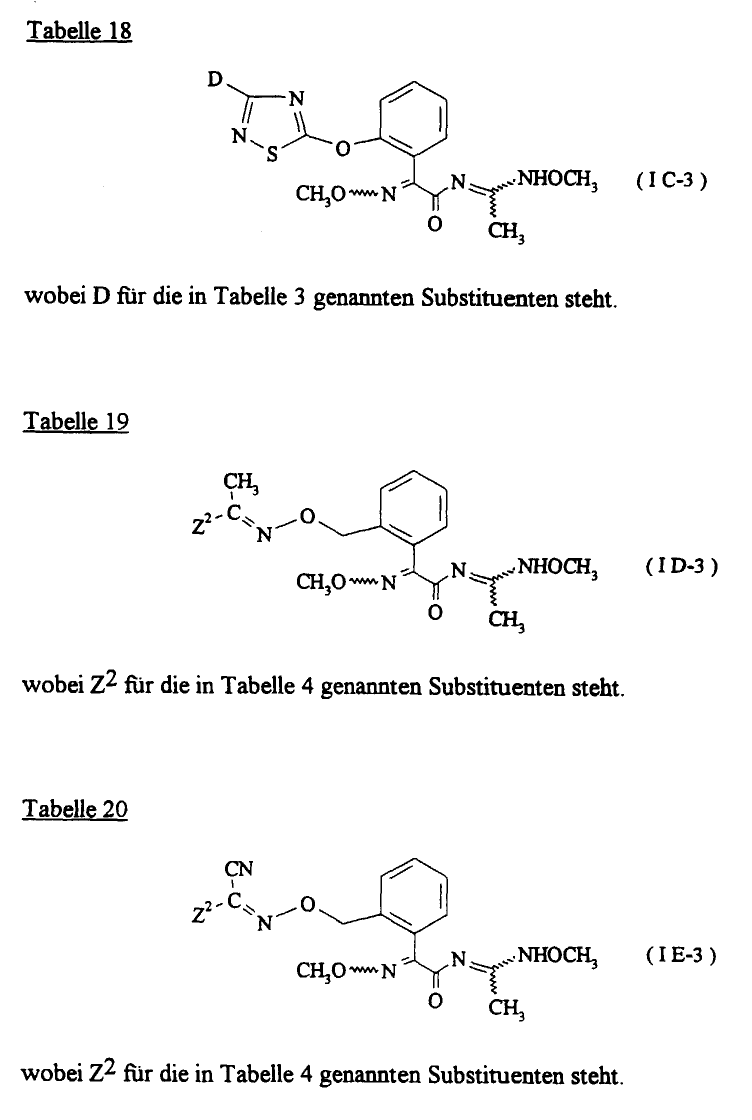

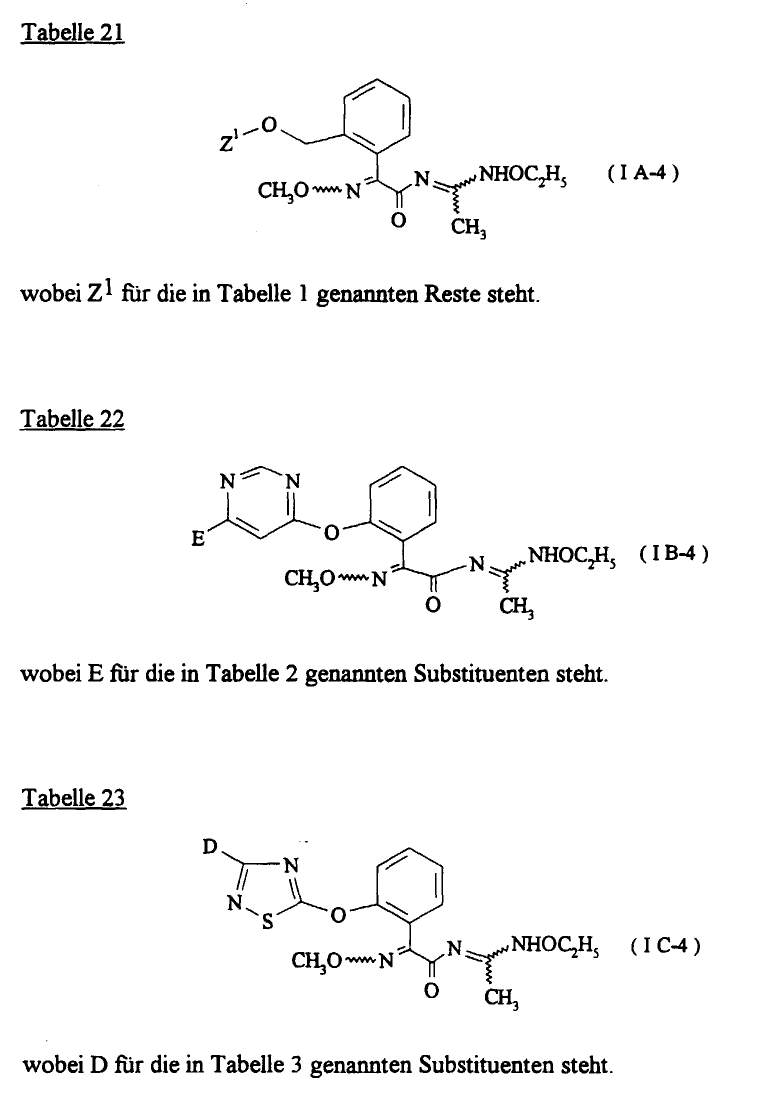

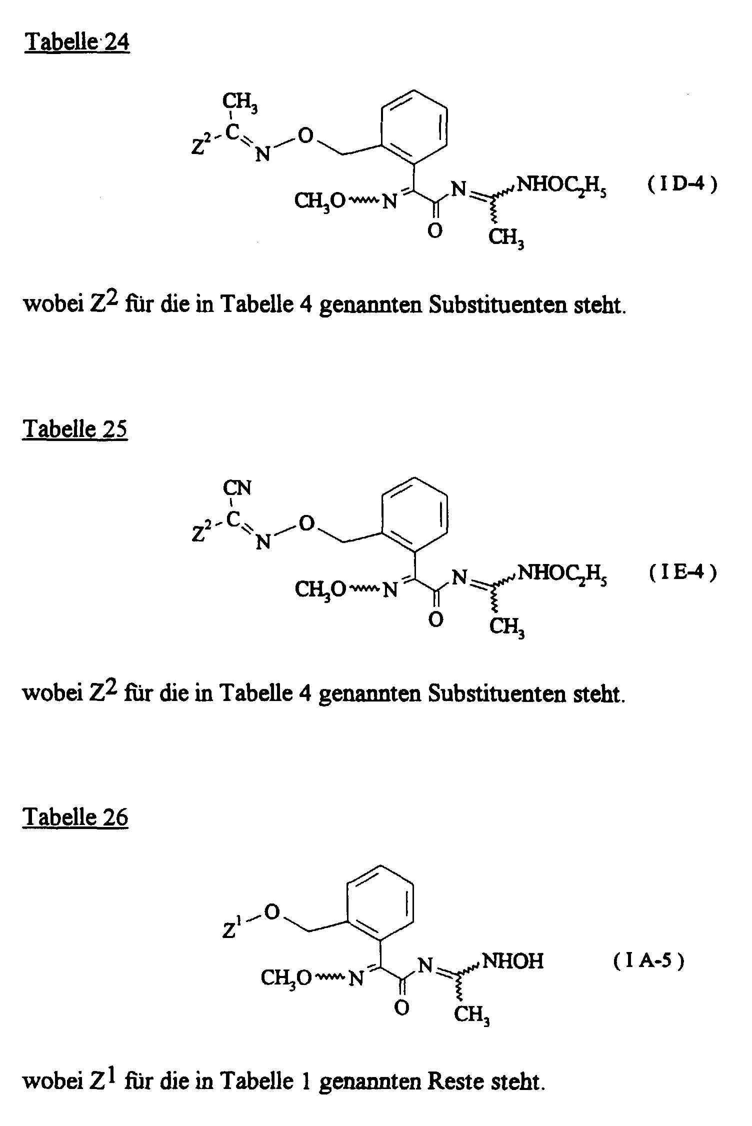

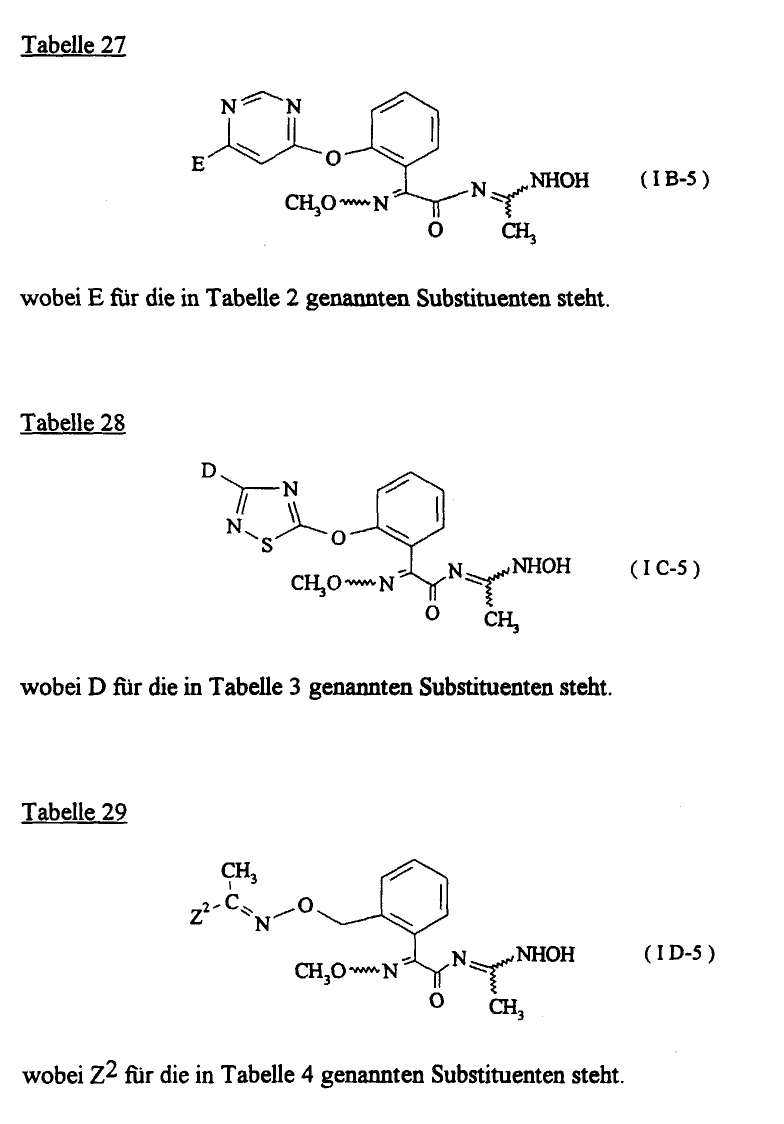

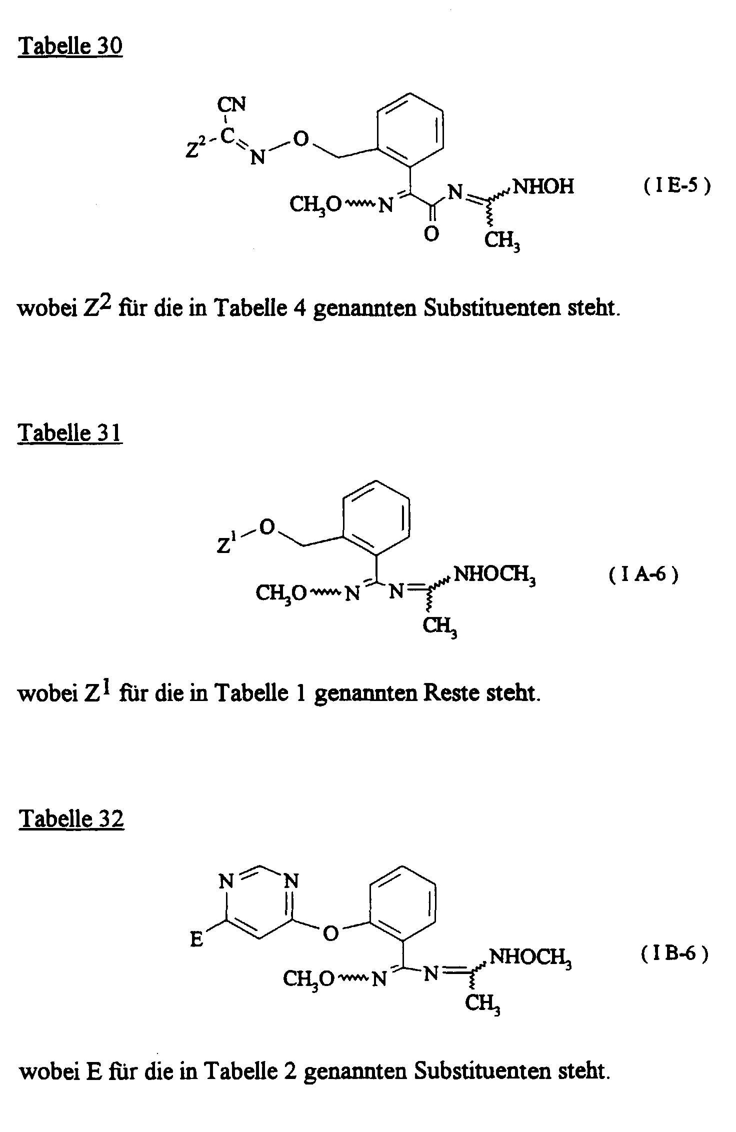

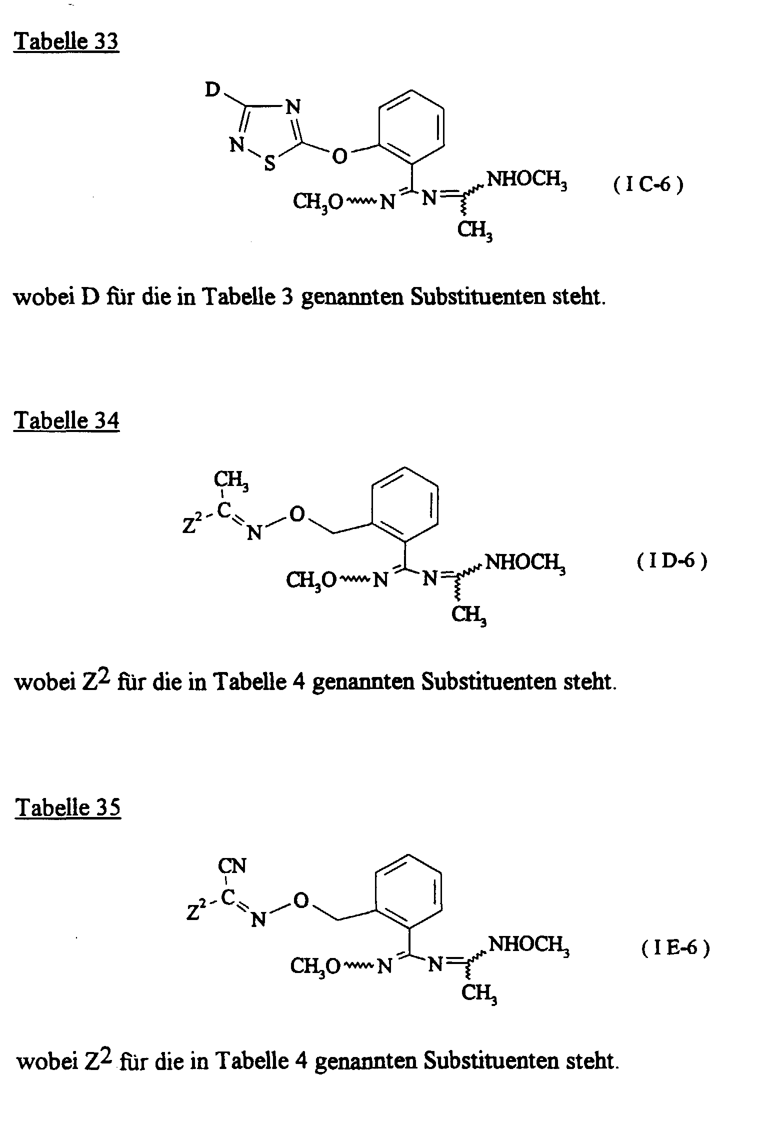

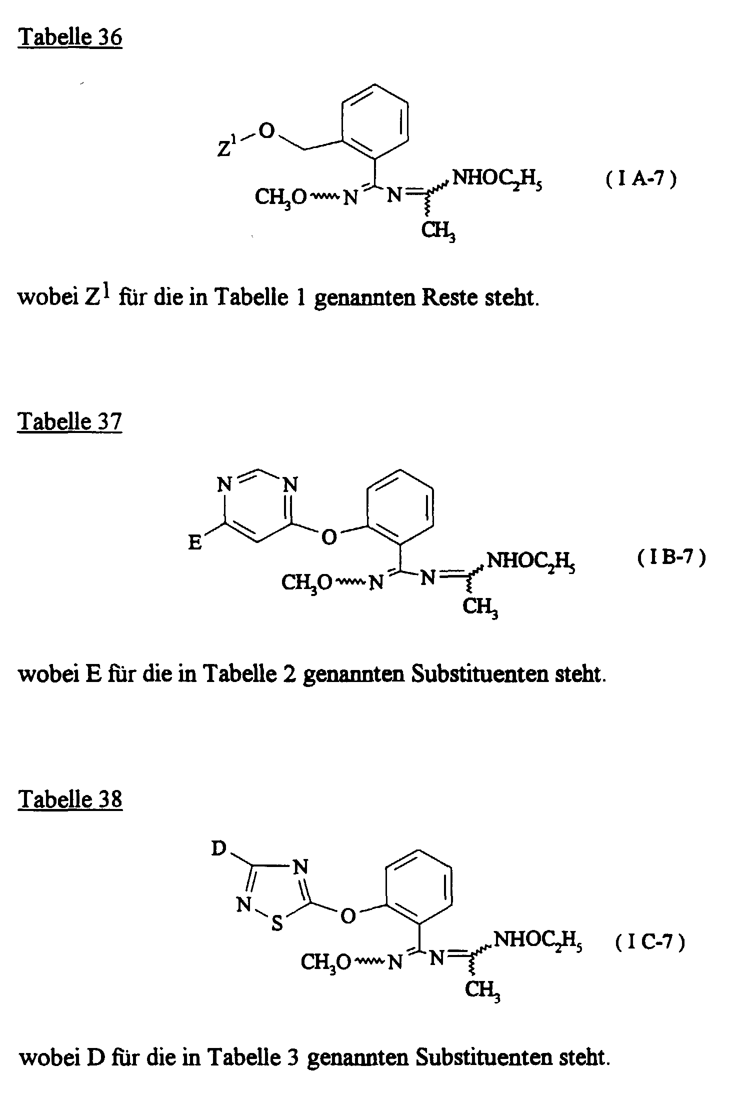

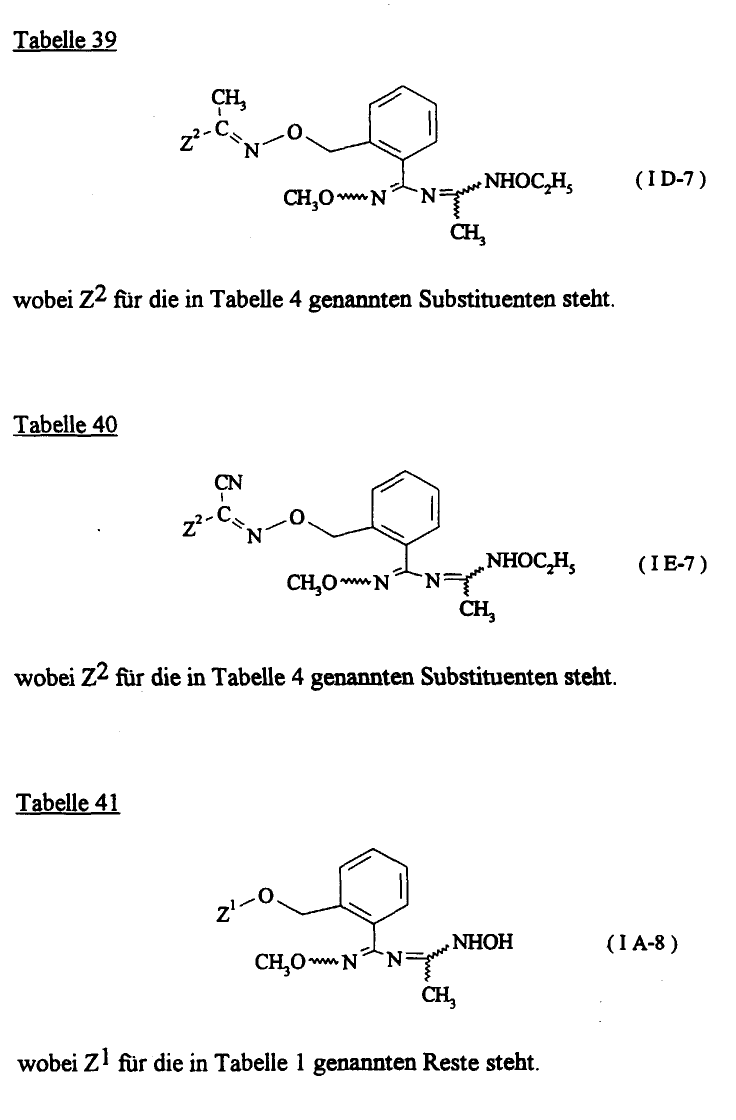

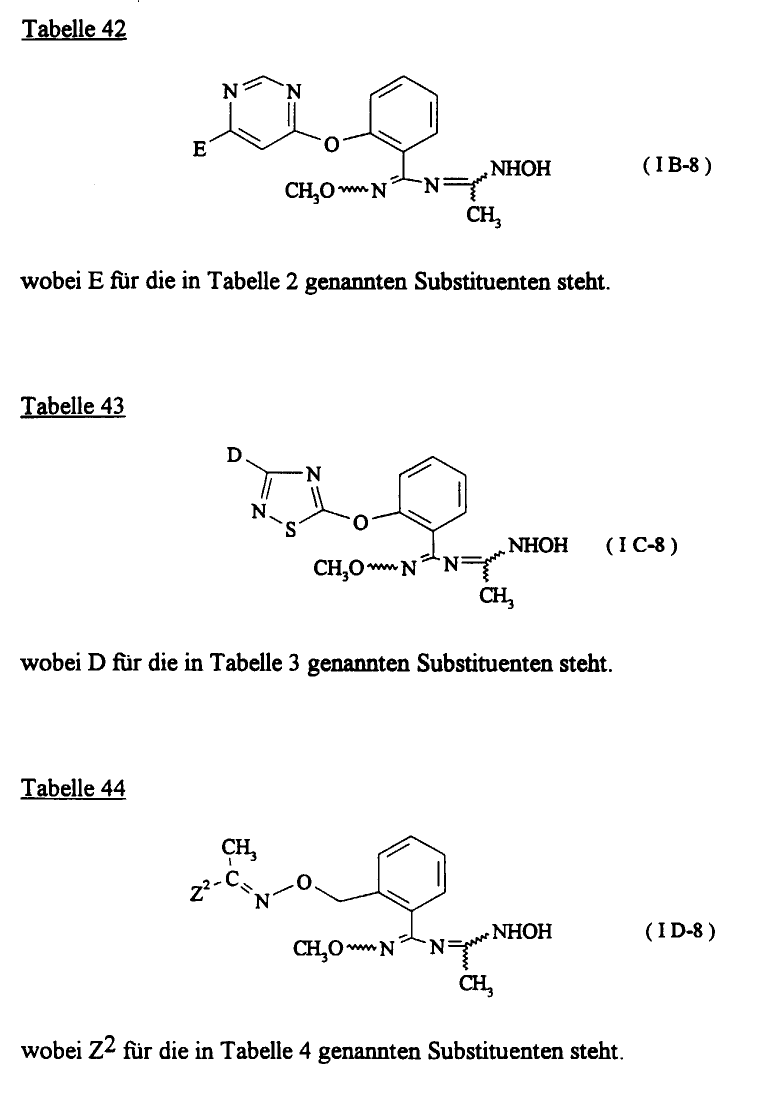

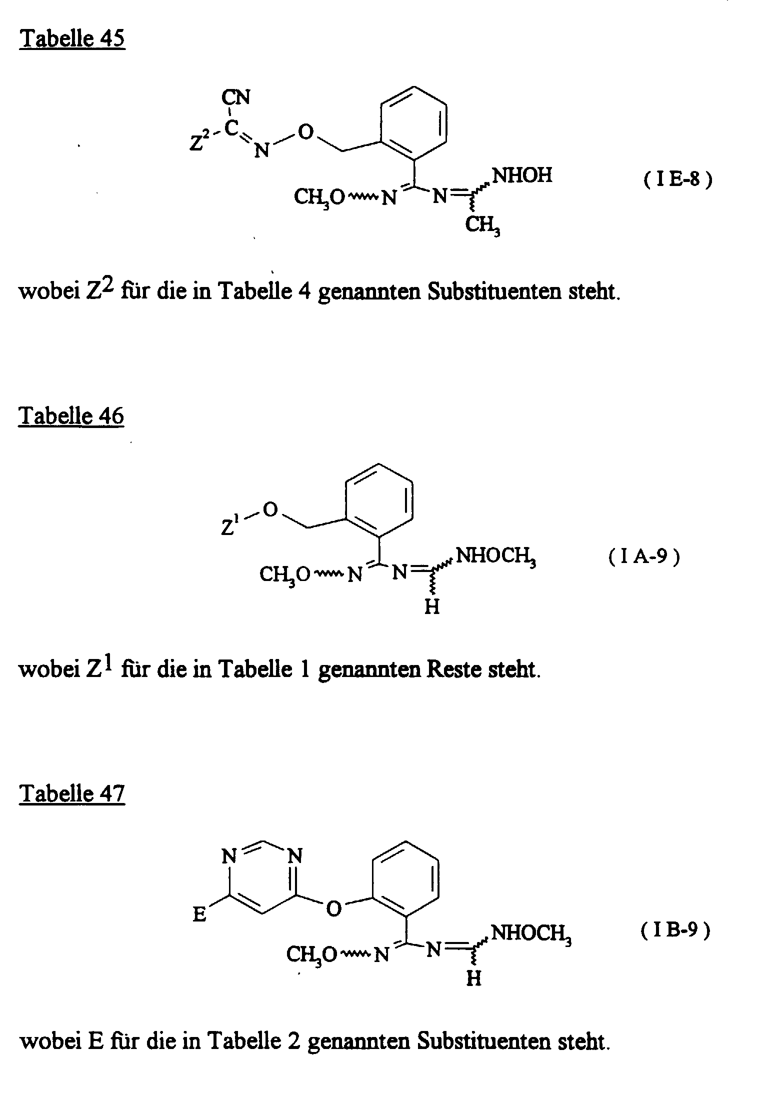

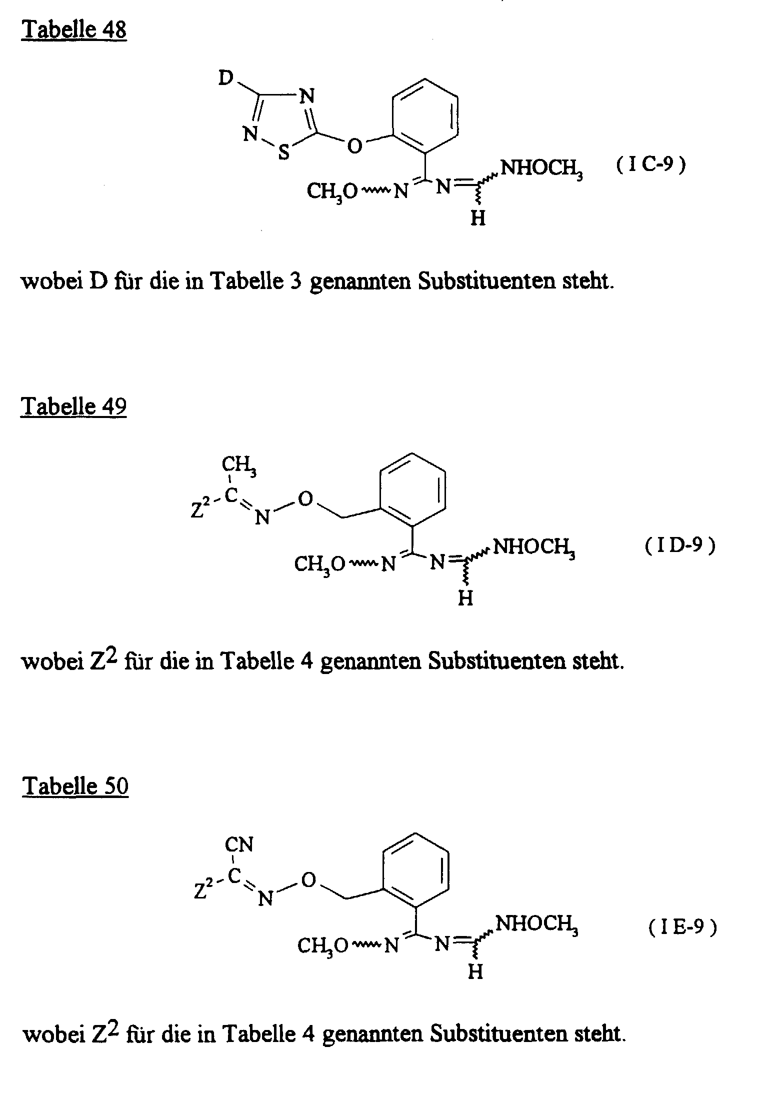

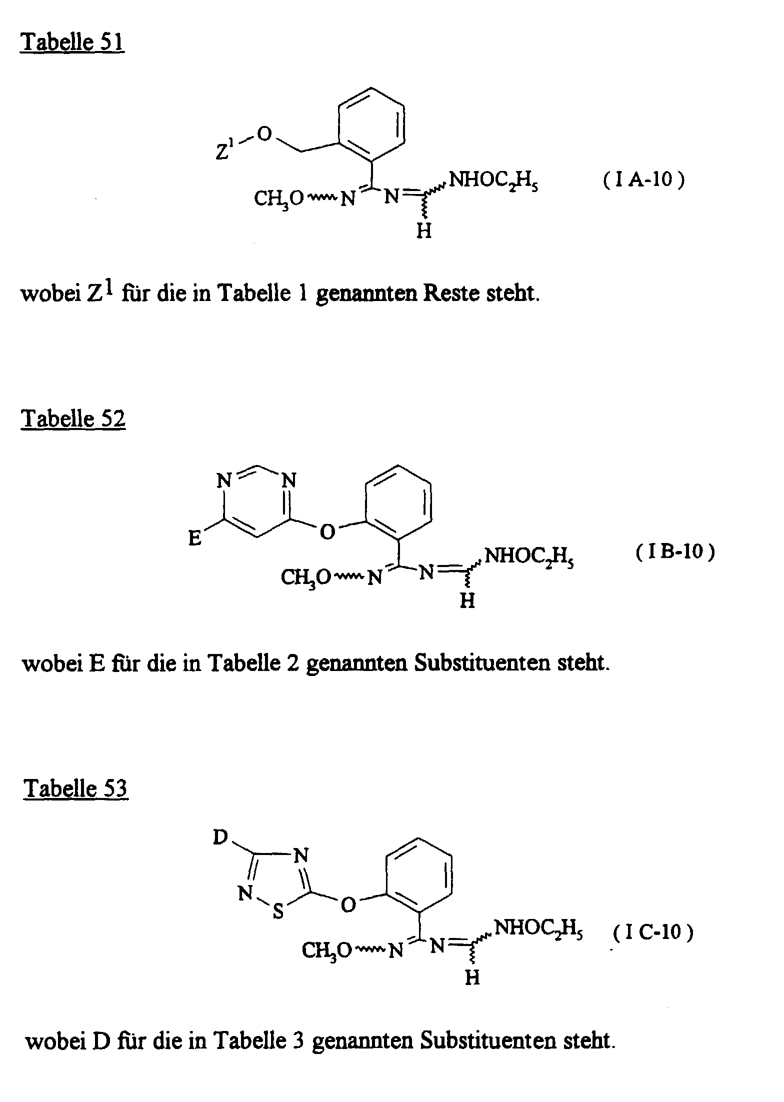

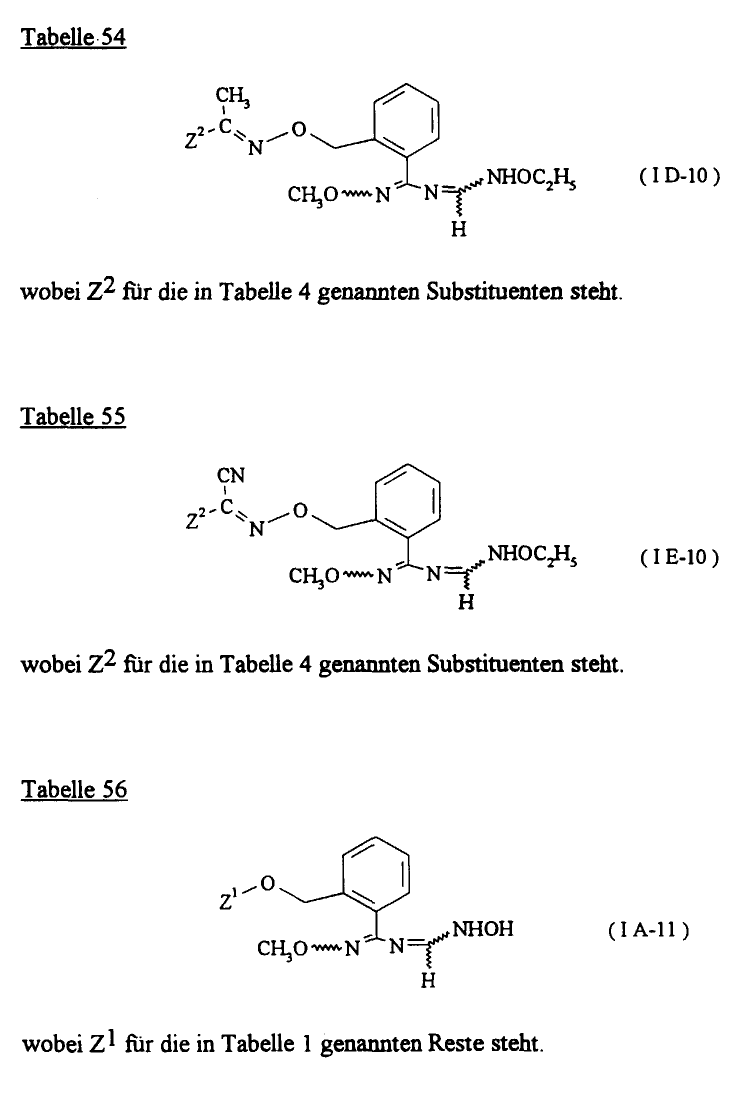

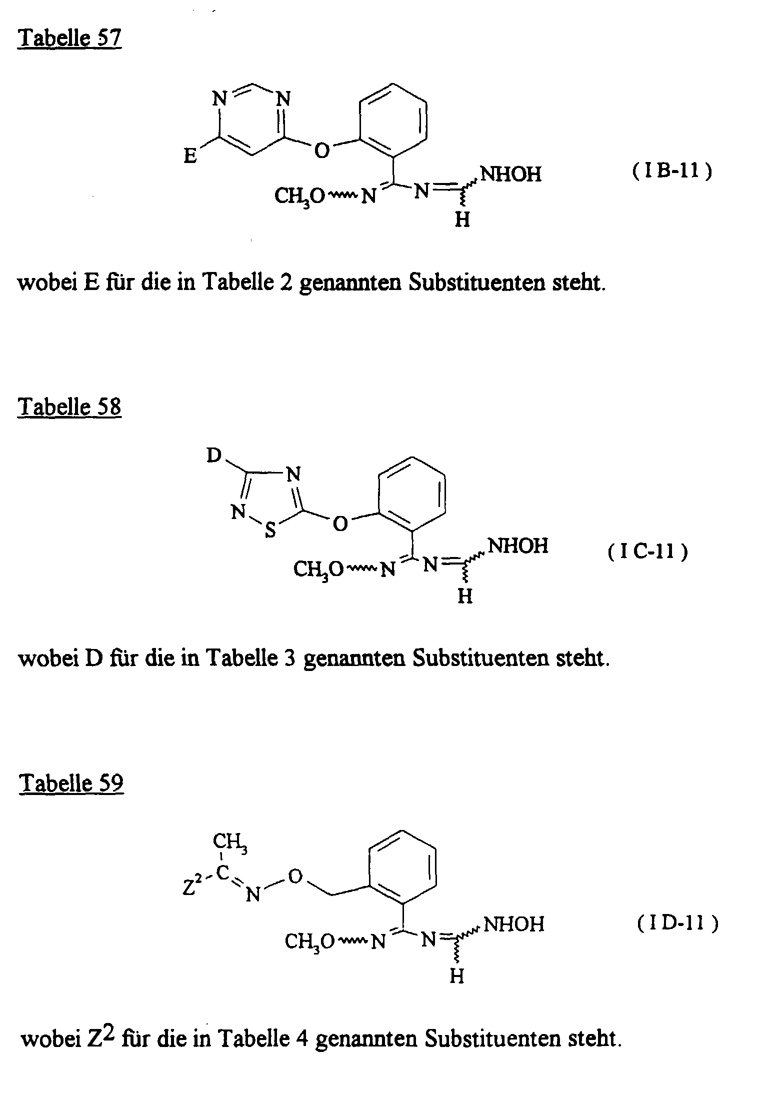

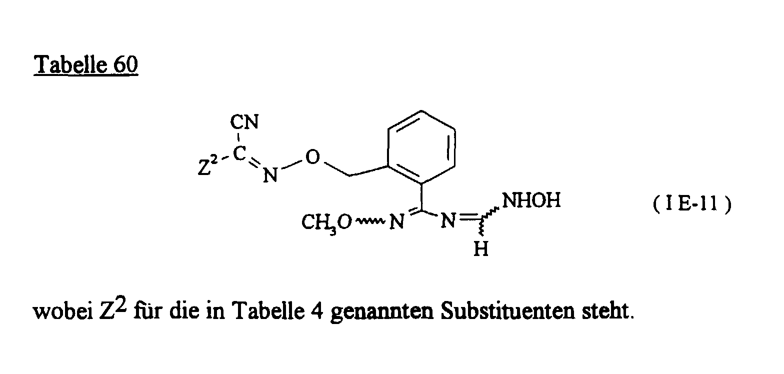

Die zur Durchführung des erfindungsgemäßen Verfahrens als Ausgangsstoffe benötigten N,N-Dialkyl-amidin-Derivate sind durch die Formel (II) allgemein definiert. In der Formel (II) haben Ar, G, R¹, R², X, Z und m vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für diese Substituenten genannt wurden.

Alk steht vorzugsweise für geradkettiges oder verzweigtes Alkyl mit 1 bis 4 Kohlenstoffatomen, besonders bevorzugt für Methyl oder Ethyl.

Die N,N-Dialkyl-amidin-Derivate der Formel (II) sind neu und ebenfalls Gegenstand dieser Erfindung. Sie werden erhalten, wenn man Oxim-Derivate der Formel (IV) 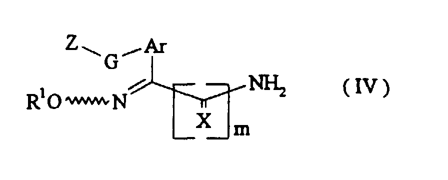 in welcher

- Ar, G, R¹, X, Z und m: die oben angegebene Bedeutung haben,
mit Aminalestern der Formel (Va) bzw. Amidacetalen der Formel (Vb) 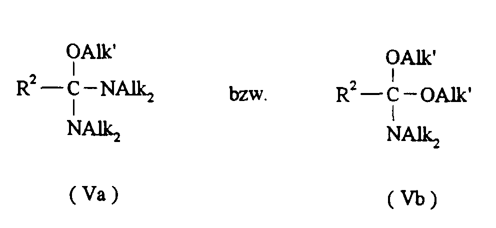 in welchen
- Alk und R²: die oben angegebene Bedeutung haben und
- Alk': für geradkettiges oder verzweigtes Alkyl mit 1 bis 4 Kohlenstoffatomen, vorzugsweise für Methyl, Ethyl, n- oder i-Propyl und n-, i-, s- oder t-Butyl steht,
gegebenenfalls in Gegenwart eines Verdünnungsmittels umsetzt.

Die Oxim-Derivate der Formel (IV) mit m = 1 sind bekannt (vgl. z.B. EP-A 0 398 692) bzw. können sie nach den dort beschriebenen Verfahren in allgemein bekannter Art und Weise erhalten werden, indem man z.B. entsprechende Ester-Derivate mit Ammoniak umsetzt (vgl. auch die Herstellungsbeispiele).

Die Oxim-Derivate der Formel (IV) mit m = 0 sind Gegenstand einer eigenen, älteren Anmeldung, die noch nicht offengelegt ist (vgl. die Deutsche Patentanmeldung P 44 08 006 vom 10.03.1994). Sie werden erhalten, indem man entsprechende Keto-Derivate der Formel (VI) 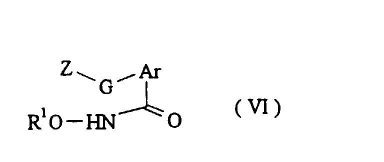 in welcher
- Ar, G, R¹ und Z: die oben angegebene Bedeutung haben,
mit einem Schwefelungsmittel, wie z.B. P₄S₁₀ oder Lawesson-Reagenz, gegebenenfalls in einem Verdünnungsmittel, wie z.B. Xylol oder Toluol, bei Temperaturen zwischen 80 und 200°C umsetzt; die erhaltenen Thion-Derivate der Formel (VII) 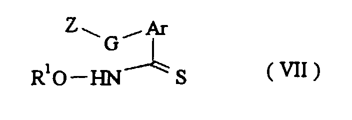 in welcher
- Ar, G, R¹ und Z: die oben angegebene Bedeutung haben,
in üblicher Weise alkyliert, wie z.B. mit Dimethylsulfat oder Methyljodid und die so erhaltenen alkylierten Derivate der Formel (VIII) 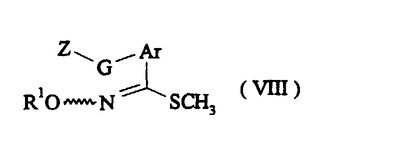 in welcher
- Ar, G, R¹ und Z: die oben angegebene Bedeutung haben,
in üblicher Weise mittels Ammoniak umsetzt (vgl. auch die Herstellungsbeispiele).

Die Aminalester der Formel (Va) bzw. die Amidacetale der Formel (Vb) sind allgemein bekannte Verbindungen der organischen Chemie.

Die Umsetzung der Oxim-Derivate der Formel (IV) mit Aminalestern der Formel (Va) bzw. Amidacetalen der Formel (Vb) wird gegebenenfalls in Gegenwart eines Verdünnungsmittels durchgeführt. Hierfür kommen vorzugsweise dipolar aprotische Lösungsmittel infrage, wie beispielsweise Dimethylformamid, Diethylformamid, N-Methylformanilid und Dimethylacetamid.

Die Reaktionstemperaturen können bei der Durchführung der Umsetzung der Oxim-Derivate der Formel (IV) mit Aminalestern der Formel (Va) bzw. Amidacetalen der Formel (Vb) in einem größeren Bereich variiert werden. Im allgemeinen arbeitet man bei Temperaturen zwischen 20 und 140°C, vorzugsweise bei Temperaturen zwischen 20 und 40°C für die Umsetzung mit Aminalestern und zwischen 100 und 130°C für die Umsetzung mit Amidacetalen.

Zur Durchführung der Umsetzung der Oxim-Derivate der Formel (IV) mit Aminalestern der Formel (Va) bzw. Amidacetalen der Formel (Vb) setzt man je Mol an Oxim-Derivat der Formel (IV) im allgemeinen 1 bis 5 Mol, vorzugsweise 1 bis 3 Mol an Aminalester der Formel (Va) bzw. Amidacetal der Formel (Vb) ein.

Die außerdem zur Durchführung des erfindungsgemäßen Verfahrens als Ausgangsstoffe benötigten Hydroxylamin-Derivate sind durch die Formel (III) allgemein definiert. In der Formel (III) hat R³ vorzugsweise bzw. insbesondere bevorzugt diejenigen Bedeutungen, die bereits oben im Zusammenhang mit der Beschreibung der Verbindungen der Formel (I) als bevorzugt bzw. insbesondere bevorzugt für diesen Substituenten genannt wurden.

Die Hydroxylamin-Derivate der Formel (III) sowie deren Säureadditionssalze, wie beispielsweise Hydrochloride und Hydroacetate sind allgemein bekannte Verbindungen der organischen Chemie.

Als Verdünnungsmittel zur Durchführung des erfindungsgemäßen Verfahrens kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäureamid, Ester, wie Essigsäureethylester oder Sulfoxide, wie Dimethylsulfoxid, Alkohole, wie Methanol oder Ethanol oder basische Lösungsmittel wie Pyridin oder Triethylamin.

Das erfindungsgemäß Verfahren wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -alkoholate, -acetate, -carbonate oder -hydrogencarbonate, wie beispielsweise Natriumhydroxid, Natriummethylat, Natriumethylat, Kalium-t-butylat, Natriumacetat, Kaluimacetat, Natriumcarbonat, Kaliumcarbonat oder Natriumhydrogencarbonat oder auch tertiäre Amine, wie beispielsweise Triethylamin, N,N-Deimethylanilin, Pyridin, N,N-Dimethylaminopyridin, Diazabicyclooctan (DABCO), Diazabicyclononen (DBN) oder Diazabicycloundecen (DBU). Auch saure Reaktionshilfsmittel wie beispielsweise p-Toluolsulfonsäure sind gegebenenfalls von Vorteil.

Die Reaktionstemperaturen können bei der Durchführung des erfindungsgemäßen Verfahrens in einem größeren Bereich variiert werden. Im allgemeinen arbeitet man bei Temperaturen zwischen 0°C und + 130°C, vorzugsweise bei Temperaturen zwischen 20°C und 80°C.

Zur Durchführung des erfindungsgemäßen Verfahrens setzt man pro Mol an N-N-Dialkyl-amidin-Derivat der Formel (II) im allgemeinen 1 bis 4 Mol, vorzugsweise 1 bis 2 Mol an Hydroxylamin-Derivat der Formel (III) und gegebenenfalls 1 bis 3 Mol, vorzugsweise 1 bis 2 Mol an Reaktionshilfsmittel.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe der Formeln (I) und (II) weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmoiophoromycetes, Oomycetes, Chytridiomycetes, Zygomycetes, Ascomycetes, Basisdiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform: Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botryris cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen

Podosphaera-Arten, zur Bekämpfung von Getreidekrankheiten, wie beispielsweise gegen Erysiphe-, Leptosphearia-, Pyrenophora- und Fusarium-Arten, oder zur Bekämpfung von Reiskrankheiten, wie beispielsweise gegen Pyricularia oryzae eingesetzt werden

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthalene, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cylcohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden; Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe werden als solche oder in ihren Formulierungen auch in Mischung mit bekannten Fungiziden, Bakteriziden, Akariziden, Nematiziden oder Insektiziden verwendet, um so z.B. das Wirkungsspektrum zu verbreitern oder Resistenzentwicklungen vorzubeugen. In vielen Fällen treten dabei synergistische Effekte auf.

### Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazok Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Maucopper, Mancozeb, Maneb, Mepanipyrum, Mepronil, Metalaxyl, Metconazol, Methasulfocalb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin;
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin;
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin Clocythrin, Clofentezin, Cyanophos, Cycloprothrin, Cyfluthrin, Cyhalothrin; Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Ferbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat, Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel (I-1)

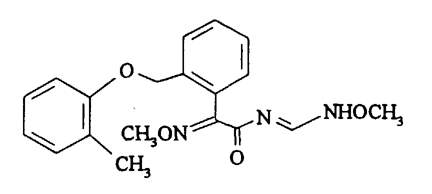

Eine Mischung aus 3,5 g (0,01 Mol) ,-[2-(2-Methylphenoxymethyl)-phenyl]-,-methoximino-N-(dimethylaminomethyliden)-acetamid (vgl. Bsp. II-1), 1,0 g (0,012 Mol) O-Methylhydroxylammoniumchlorid und 1,0 g (0,012 Mol) Natriumacetat in 25 mi Methanol wird 24 Stunden bei Raumtemperatur gerührt. Nach Einengen wird der Rückstand mit Essigester verrührt, die organische Phase mit Wasser gewaschen, über Natriumsulfat getrocknet und eingeengt. Der Rückstand wird chromatographisch an Kieselgel (Laufmittel: Cyclohexan/Essigester = 2/1) gereinigt.

Man erhalt 1,2 g (34% der Theorie) ,-[2-(2-Methylphenoxymethyl)-phenyl]-,-methoximino-N-(methoxyaminomethyliden)-acetamid.
¹HNMR (CDCl₃) /(ppm) = 3,95 (s, 3H).

Analog Beispiel (I-1) sowie entsprechend der allgemeinen Beschreibung des erfindungsgemäßen Verfahrens werden die in der nachstehenden Tabelle aufgeführten Verbindungen der Formel (I) erhalten:

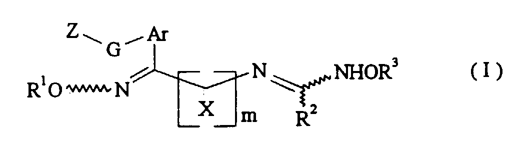

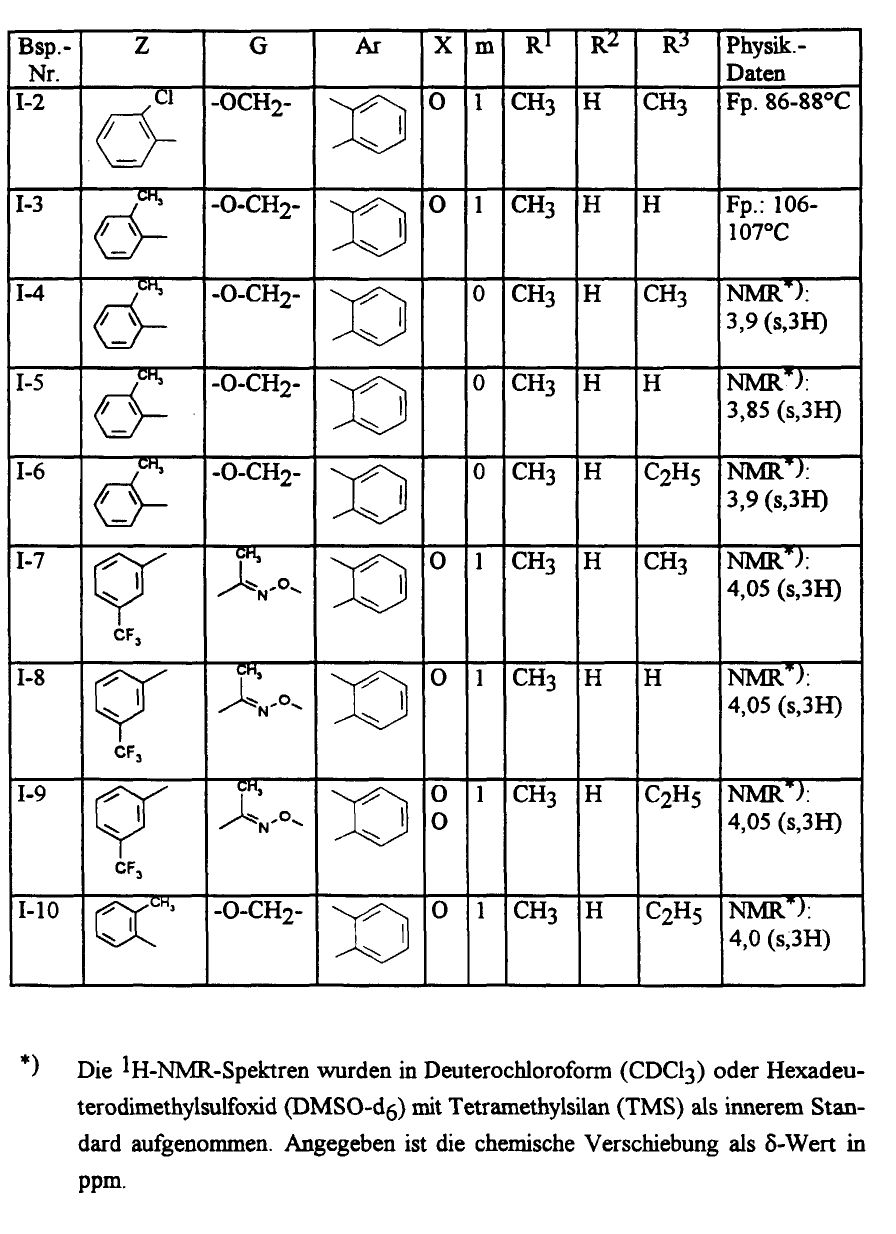

### Herstellung der Ausgangsprodukte

### Beispiel (II-1)

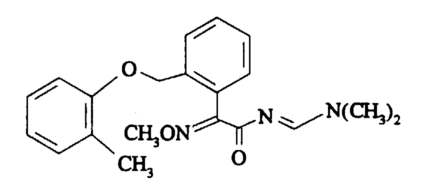

Ein Gemisch aus 4,8 g (0,016 Mol) ,-[2-(2-Methylphenoxymethyl)-phenyl]-,-methoximino-acetamid und 6,6 g (0,038 Mol) tert.-Butoxy-bis (N,N-dimethylamino)-methan (vgl. Bsp. IV-1) wird 24 Stunden bei 40°C gerührt. Danach wird auf Raumtemperatur abgekühlt und im Hochvakuum eingeengt.

Man erhalt 4,9 g (87% der Theorie) ,-[2-(2-Methylphenoxymethyl)-phenyl]-,-methoximino-N-(dimethylaminomethyliden)-acetamid.
¹HNMR (CDCl₃) /(ppm) = 4,0 (s, 3H).

### Herstellung des Vorprodukts

### Beispiel (IV-1)

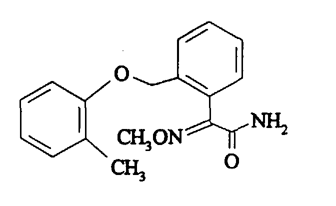

Zu 10 g (0,032 Mol) 2-(2-Methylphenoxymethyl)-,-methoximino-phenylessigsäuremethylester in 100 ml Methanol gibt man 100 ml einer 25%-igen wäßrigen Ammoniaklösung und erhitzt das Reaktionsgemisch 5 Stunden unter Rückfluß. Danach werden nochmals 100 ml einer 25%-igen wäßrigen Ammoniaklösung zugegeben und weitere 6 Stunden unter Rückfluß erhitzt. Zur Vervollständigung der Reaktion werden weitere 50 ml einer 25%-igen Ammoniaklösung hinzugegeben und das Reaktionsgemisch 8 Stunden unter Rückfluß erhitzt. Nach Abkühlung auf Raumtemperatur gibt man das Reaktionsgemisch auf Wasser, extrahiert mit Essigester, wäscht die organische Phase mit Wasser, trocknet über Natriumsulfat, engt im Vakuum ein und verrührt den Rückstand mit Diisopropylether.

Man erhalt 8,3 g (87% der Theorie) ,-[2-(2-Methylphenoxymethyl)-phenyl]-,-methoximino-acetamid vom Schmelzpunkt 93-95°C.

### Beispiel (II-2)

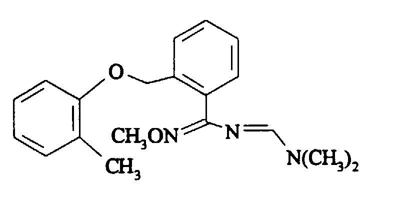

Ein Gemisch aus 0,6 g (0,022 Mol) Amino-methoximino-[2-(2-Methylphenoxymethyl)-phenyl]-methan und 5 ml tert.-Butoxy-bis (N,N-dimethylamino)-methan wird über Nacht bei Raumtemperatur gerührt. Das Reaktionsgemisch wird anschließend in Wasser gegeben, mit Essigsäureester extrahiert, die organische Phase mit Wasser gewaschen, über Natriumsulfat getrocknet und eingeengt.

Man erhalt 0,6 g (83 % der Theorie) Dimethylaminomethylidenamino-methoximino-[2-(2-Methylphenoxymethyl)-phenyl]-methan.
¹HNMR (DMSO-d₆) /(ppm) = 3,7 (s, 3H).

Analog den Beispielen (II-1) und (II-2) sowie entsprechend der allgemeinen Beschreibung werden die in der nachstehenden Tabelle aufgeführten Vorprodukte der Formel (II) erhalten:

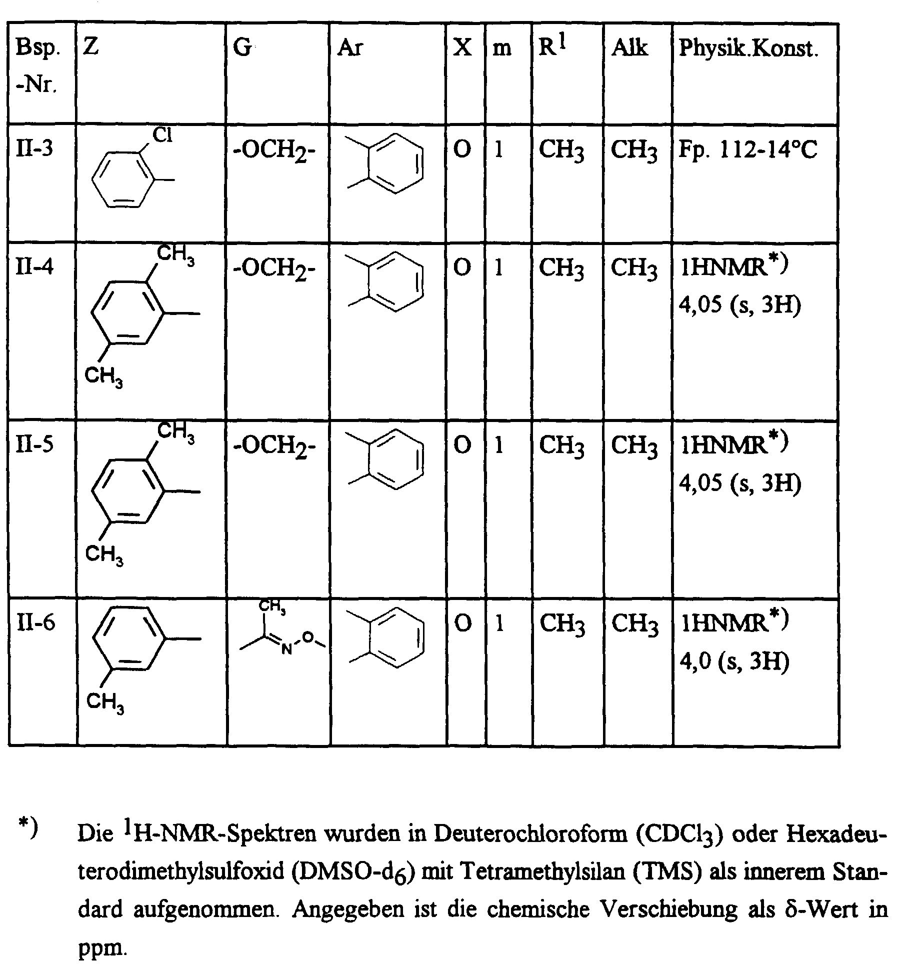

### Anwendungsbeispiele

### Beispiel A

### Erysiphe-Test (Weizen) / protectiv

- Lösungsmittel :: 10 Gewichtsteile N-Methyl-pyrrolidon
- Emulgator :: 0,6 Gewichtsteile Alkyl-Aryl-Polyglykolether

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von *Erysiphe graminis f.sp. tritici* bestäubt.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

In diesem Test zeigt die Verbindung gemäß Herstellungsbeispiel (I-1) bei einer Wirkstoffaufwandmenge von 250 g/ha einen Wirkungsgrad von 100.

### Beispiel B

### Erysiphe-Test (Weizen) / kurativ

- Losungsmittel :: 10 Gewichtsteile N-Methyl-pyrrolidon
- Emulgator :: 0,6 Gewichtsteile Alkyl-Aryl-Polyglykolether

Zur Herstellung einer zweckmäßigen Wirkstoffbereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von *Erysiphe graminis f.sp. tritici* bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

In diesem Test zeigt die Verbindung gemäß Herstellungsbeispiel (I-1) bei einer Wirkstoffaufwandmenge von 250 g/ha einen Wirkungsgrad von 100.

### Beispiel C

### Erysiphe-Test (Gerste)/ kurativ

- Lösungsmittel :: 10 Gewichtsteile N-Methyl-pyrrolidon
- Emulgator :: 0,6 Gewichtsteile Alkyl-Aryl-Polyglykolether

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von *Erysiphe graminis f.sp. hordei* bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80% aufgestellt, um die Entwicklung von Mehltaupusteln zu begünstigen.

7 Tage nach der Inokulation erfolgt die Auswertung.

In diesem Test zeigt die Verbindung gemäß Herstellungsbeispiel (I-1) bei einer Wirkstoffaufwandmenge von 250 g/ha einen Wirkungsgrad von 100.

### Beispiel D

### Podosphaera-Test (Apfel) / protektiv

- Lösungsmittel :: 4,7 Gewichtsteile Aceton
- Emulgator :: 0,3 Gewichtsteile Alkyl-Aryl-Polyglykolether

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüffing auf protektive Wirksamkeit bespritzt man junge Pflanzen mit der Wrikstoffzubereitung bis zur Tropfnässe. Nach Antrocknen des Spritzbelages werden die Pflanzen durch Bestäuben mit Konidien des Apfelmehltauerregers *Podosphaera leucotricha* inokuliert.

Die Pflanzen werden dann im Gewächshaus bei 23°C und einer relativen Luftfeuchtigkeit von ca. 70% aufgestellt.

10 Tage nach der Inokulation erfolgt die Auswertung.

In diesem Test zeigt die Verbindung gemäß Herstellungsbeispiel (I-1) bei einer Wirkstoffkonzentration von 20 ppm einen Wirkungsgrad von 89%.

### Beispiel E

### Plasmopara-Test (Rebe) / protektiv

- Lösungsmittel :: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen mit einer wäßrigen Sporensuspension von Plasmopara viticola inokuliert und verbleiben dann 1 Tag in einer Feuchtkammer bei 20 bis 22°C und 100 % relativer Luftfeuchtigkeit. Anschließend werden die Pflanzen 5 Tage im Gewächshaus bei 21°C und 90 % Luftfeuchtigkeit aufgestellt. Die Pflanzen werden dann angefeuchtet und 1 Tag in eine Feuchtekammer gestellt.

6 Tage nach der Inokulation erfolgt die Auswertung.

Die Verbindung gemäß Herstellungsbeispiel I-10 zeigt bei einer Auswandmenge von 100 ppm 84 % Wirkungsgrad.

### Beispiel F

### Podosphaera-Test (Apfel) (protektiv)

- Lösungsmittel:: 4,7 Gewichtsteile Aceton
- Emulgator:: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung besprüht. Nach Antrocknen des Spritzbelages werden die Pflanzen durch Bestäuben mit Konidien des Apfelmehltauerregers Podosphaera Leucotricha inokuliert.

Die Pflanzen werden dann im Gewächshaus bei 23°C und einer relativen Luftfeuchtigkeit von ca. 70% aufgestellt.

10 Tage nach der Inokulation erfolgt die Auswertung.

Die Verbindungen gemäß Herstellungsbeispielen I-4, I-52 und I-53 zeigen bei einer Wirkstoffkonzentration von 100 ppm bis zu 98 %ige Wirkungsgrade.

### Beispiel G

### Erysiphe-Test (Gerste) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Die Verbindungen gemäß Herstellungsbeispielen I-3 und II-6 zeigen bei einer Aufwandmenge von 250 g/ha einen 100 %igen Wirkungsgrad.

### Beispiel H

### Erysiphe-Test (Gerste) / protektiv

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge.

Nach Antrocknen des Spritzbelages werden die Pflanzen mit Sporen von Erysiphe graminis f.sp. tritici bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Die Verbindung gemäß Herstellungsbeispiel I-3 zeigt bei einer Aufwandmenge von 250 g/ha einen 100 %igen Wirkungsgrad.

### Beispiel I

### Erysiphe-Test (Gerste) / kurativ

- Lösungsmittel:: 10 Gewichtsteile N-Methyl-pyrrolidon
- Emulgator:: 0,6 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f. sp. hordei bestäubt. 48 Stunden nach der Inokulation werden die Pflanzen mit der Wirkstoffzubereitung in der angegebenen Aufwandmenge besprüht.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Verbindungen gemäß Herstellungsbeispiel II-1 zeigt bei einer Aufwandmenge von 250 g/ha einen 100 %igen Wirkungsgrad.

## Patentansprüche

1. N-Alkoxy-amidin-Derivate der allgemeinen Formel (I), 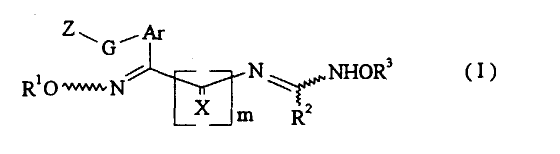 in welcher
Ar für jeweils gegebenenfalls substituiertes Arylen oder Heteroarylen steht;
G für eine Einfachbindung für Sauerstoff, für jeweils gegebenenfalls durch Halogen, Hydroxy, Alkyl, Halogenalkyl oder Cycloalkyl substituiertes Alkandiyl, Alkendiyl, Oxaalkendiyl, Alkindiyl oder eine der nachstehenden Gruppierungen steht
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂-oder
-N=N-C(R⁴)=N-O-,
wobei
n für die Zahlen 0, 1 oder 2 steht,
Q für Sauerstoff oder Schwefel steht,
R¹ für Wasserstoff oder Alkyl steht;
R² für Wasserstoff oder Alkyl steht;
R³ für Wasserstoff oder Alkyl steht;
R⁴ für Wasserstoff, Cyano oder jeweils gegebenenfalls substituiertes Alkyl, Alkoxy, Alkylthio, Alkylamino, Dialkylamino oder Cycloalkyl steht, und
R⁵ für Wasserstoff, Hydroxy, Cyano oder jeweils gegebenenfalls substituiertes Alkyl, Alkoxy oder Cycloalkyl steht;
X für Sauerstoff oder Schwefel steht;
m für die Zahlen 0 oder 1 steht und
Z für jeweils gegebenenfalls substituiertes Alkyl, Alkenyl, Alkinyl, Cycloalkyl, Aryl oder Heterocyclyl steht.

2. Verbindungen der Formel (I) nach Anspruch 1, in welcher
Ar für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten vorzugsweise aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen,
G für eine Einfachbindung, für Sauerstoff, für jeweils gegebenenfalls durch Halogen, Hydroxy, C₁-C₄-Alkyl C₁-C₄-Halogenalkyl oder C₃-C₆-Cycloalkyl substituiertes Alkandiyl, Alkendiyl, Oxaalkendiyl, Alkindiyl mit jeweils bis zu 4 Kohlenstoffatomen oder eine der nachstehenden Gruppierungen steht
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- oder -N=N-C(R⁴)=N-=-,
wobei
n für die Zahlen 0, 1 oder 2 steht,
Q für Sauerstoff oder Schwefel steht,
R⁴ für Wasserstoff, Cyano, für gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlerstoffatomen steht, und
R⁵ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C1-C4-Alkoxy-carbonyl substituiertes Cycloalkyl mit 3 bis 6 Kohlenstoffatomen steht.
R¹ für Wasserstoff oder geradkettiges oder verzweigtes Alkyl mit 1 bis 4 Kohlenstoffatomen steht.
R² für Wasserstoff oder geradkettiges oder verzweigtes Alkyl mit 1 bis 4 Kohlenstoffatomen steht.
R³ für Wasserstoff oder geradkettiges oder verzweigtes Alkyl mit 1 bis 4 Kohlenstoffatomen steht.
X für Sauerstoff oder Schwefel steht.
m für die Zahlen 0 oder 1 steht und
Z für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen, für jeweils gegebenenfalls substituiertes Phenyl, Naphthyl oder (gegebenenfalls benzannelliertes) Heterocyclyl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten vorzugsweise aus der nachstehenden Aufzählung ausgewählt sind:
Sauerstoff (als Ersatz für zwei geminale Wasserstoffatome), Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano, Nitro Carboxy, Carbamoyl und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen und/oder Alkylcarbonyl oder Alkoxycarbonyl mit jeweils bis zu 5 Kohlenstoffatomen substituiertes Phenyl, Phenoxy, Benzyl, Benzyloxy, Phenylethyl oder Phenylethyloxy.

3. Verbindungen der Formel (I) gemäß Anspruch 1, in welcher
Ar für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl, Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio, Methylsulfinyl oder Methylsulfonyl.
G für eine Einfachbindung, für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q oder -N(R⁵)-CQ-Q-CH₂- steht,
wobei
n für die Zahlen 0, 1 oder 2 steht,
Q für Sauerstoff oder Schwefel steht,
R⁴ für Wasserstoff, Cyano, für gegebenenfalls durch Fluor, Chlor, Cyano, Methoxy oder Ethoxy substituiertes Methyl, Ethyl, n- oder i-Propyl, n-, i-oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R⁵ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht.
R¹ für Wasserstoff, Methyl, Ethyl, n- oder i-Propyl sowie n-, i-, s- oder t-Butyl steht.
R² für Wasserstoff, Methyl, Ethyl, n- oder i-Propyl sowie n-, i-, s- oder t-Butyl steht.
R³ für Wasserstoff, Methyl, Ethyl, n- oder i-Propyl sowie n-, i-, s- oder t-Butyl steht.
X für Sauerstoff oder Schwefel steht.
m für die Zahlen 0 oder 1 steht und
Z für gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl für jeweils gegebenenfalls durch Fluor, Chlor oder Brom substituiertes Allyl, Crotonyl, 1-Methyl-allyl, Propargyl oder 1-Methyl-propargyl, für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl, für jeweils gegebenenfalls substituiertes Phenyl, Naphthyl, Furyl, Tetrahydrofuryl, Benzofuryl, Tetrahydropyranyl, Thienyl, Benzothienyl, Pyrrolyl, Dihydropyrrolyl, Tetrahydropyrrolyl, Benzopyrrolyl, Benzodihydropyrrolyl, Oxazolyl, Benzoxazolyl, Isoxazolyl, Thiazolyl, Benzthiazolyl, Isothiazolyl, Imidazolyl, Benzimidazolyl, Oxadiazolyl, Thiadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Sauerstoff (als Ersatz für zwei geminale Wasserstoffatome), Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Nitro, Carboxy, Carbamoyl, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Acetyl, Methoxycarbonyl oder Ethoxycarbonyl substituiertes Phenyl, Phenoxy, Benzyl oder Benzyloxy.

4. Verbindungen der Formel (I) gemäß Anspruch 1, in welcher
Ar für ortho-Phenylen, Pyridin-2,3-diyl oder Thiophen-2,3-diyl steht,
G für Sauerstoff, Methylen oder eine der nachstehenden Gruppierungen
-CH₂-O-, -O-CH₂-, -S(O)ₙ-, -CH₂-S(O)ₙ-, S(O)ₙ-CH₂-, -C(R⁴)=N-O-, -O-N=C(R⁴)-, -C(R⁴)=N-O-CH₂-, -N(R⁵)- oder -CH₂-O-N=C(R⁴)- steht,
wobei
n für die Zahlen 0, 1 oder 2 steht,
R⁴ für Wasserstoff, Methyl oder Ethyl steht und
R⁵ für Wasserstoff, Methyl oder Ethyl steht.
R¹ für Methyl steht.
R² für Wasserstoff oder Methyl steht.
R³ für Wasserstoff oder Methyl steht.
X für Sauerstoff steht.
m für die Zahlen 0 oder 1 steht und
Z für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten vorzugsweise aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n-oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

5. Verfahren zur Herstellung von N-Alkoxy-amidin-Derivaten der allgemeinen Formel (I), dadurch gekennzeichnet, daß man N-N-Dialkyl-amidin-Derivate der Formel (II) 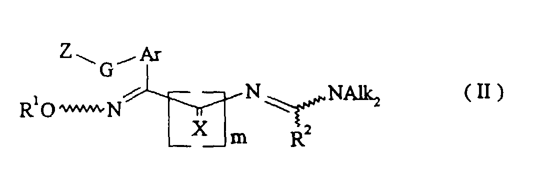 in welcher
Ar, G, R¹, R², X, Z und m die in Anspruch 1 angegebene Bedeutung haben
und
Alk für Alkyl steht,
mit Hydroxylamin-Derivaten der allgemeinen Formel (III)
H₂N―O―R³ (III)
in welcher
R³ die in Anspruch 1 angegebene Bedeutung hat,
oder mit deren Säureadditionssalzen in Gegenwart eines Verdünnungsmittels und gegebenenfalls in Gegenwart eines Reaktionshilfsmittels umsetzt.

6. N,N-Dialkyl-amidin-Derivate der Formel (II) 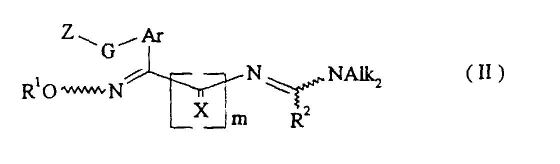 in welcher
Ar, G, R¹, R², X, Z und m die in Anspruch 1 angegebene Bedeutung haben und
Alk für Alkyl steht.

7. Schädlingsbekämpfungsmittel, gekennzeichnet durch einen Gehalt an mindestens einer Verbindung der Formeln (I) bzw. (II) nach den Ansprüchen 1 und 6.

8. Verfahren zur Bekämpfung von Schädlingen, dadurch gekennzeichnet, daß man Verbindungen der Formeln (I) bzw. (II) nach Anspruch 1 auf Schädlinge und/oder ihren Lebensraum einwirken läßt.

9. Verwendung von Verbindungen der Formeln (I) und (II) nach den Ansprüchen 1 bis 6 zur Bekämpfung von Schädlingen.

10. Verfahren zur Herstellung von Schädlingsbekämpfungsmitteln, dadurch gekennzeichnet, daß man Verbindungen der Formeln (I) bzw. (II) nach den Ansprüchen 1 bis 6 mit Streckmitteln und/oder oberflächenaktiven Mitteln vermischt.

11. Verfahren zur Herstellung von N,N-Dialkyl-amidin-Derivaten der Formel (II) nach Anspruch 6, dadurch gekennzeichnet, daß man Oxim-Derivate der Formel (IV) 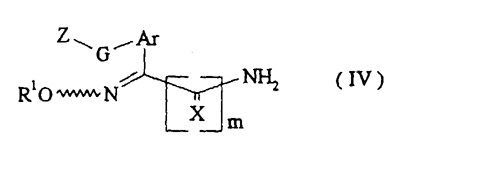 in welcher
Ar, G, R¹, X, Z und m die in Anspruch 6 angegebene Bedeutung haben,
mit Aminalestern der Formel (Va) bzw. Amidacetalen der Formel (Vb) 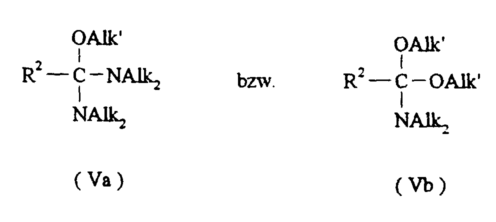 in welchen
Alk und R² die in Anspruch 6 angegebene Bedeutung haben und
Alk' für geradkettiges oder verzweigtes Alkyl mit 1 bis 4 Kohlenstoffatomen, vorzugsweise für Methyl, Ethyl, n- oder i-Propyl und n-, i-, s- oder t-Butyl steht,
gegebenenfalls in Gegenwart eines Verdünnungsmittels umsetzt.

## Claims

1. N-Alkoxy-amidine derivatives of the general formula (I), 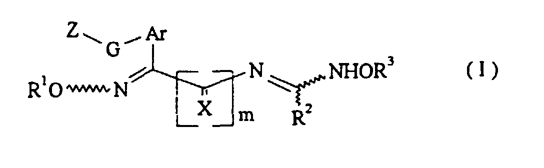 in which
Ar represents in each case optionally substituted arylene or heteroarylene;
G represents a single bond, represents oxygen, represents alkanediyl, alkenediyl, oxaalkenediyl or alkinediyl which are in each case optionally substituted by halogen, hydroxyl, alkyl, halogenoalkyl or cycloalkyl, or represents one of the following groupings
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- or -N=N-C(R⁴)=N-O-,
where
n represents the numbers 0, 1 or 2,
Q represents oxygen or sulphur,
R¹ represents hydrogen or alkyl;
R² represents hydrogen or alkyl;
R³ represents hydrogen or alkyl;
R⁴ represents hydrogen, cyano or in each case optionally substituted alkyl, alkoxy, alkylthio, alkylamino, dialkylamino or cycloalkyl, and
R⁵ represents hydrogen, hydroxyl, cyano or in each case optionally substituted alkyl, alkoxy or cycloalkyl;
X represents oxygen or sulphur;
m represents the numbers 0 or 1, and
Z represents in each case optionally substituted alkyl, alkenyl, alkinyl, cycloalkyl, aryl or heterocyclyl.

2. Compounds of the formula (I) according to Claim 1, in which
Ar represents in each case optionally substituted phenylene or naphthylene, or represents heteroarylene having 5 or 6 ring members of which at least one represents oxygen, sulphur or nitrogen and, where appropriate, one or two further members represent nitrogen, with the possible substituents preferably being selected from the following list:
halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl having in each case from 1 to 6 carbon atoms, in each case straight-chain or branched alkenyl, alkenyloxy or alkinyloxy having in each case from 2 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched halogenoalkenyl or halogenoalkenyloxy having in each case from 2 to 6 carbon atoms and from 1 to 13 identical or different halogen atoms in each case straight-chain or branched alkylamino, dialkylamino, alkylcarbonyl, alkylcarbonyloxy, alkoxycarbonyl, alkylsulphonyloxy, hydroxyiminoalkyl or alkoxyiminoalkyl having in each case from 1 to 6 carbon atoms in the individual alkyl moieties, or in each case doubly linked alkylene or dioxyalkylene which in each case have from 1 to 6 carbon atoms and which are in each case optionally substituted identically or differently, once or more than once, by halogen and/or straight-chain or branched alkyl having from 1 to 4 carbon atoms and/or straight-chain or branched halogenoalkyl having from 1 to 4 carbon atoms and from 1 to 9 identical or different halogen atoms,
G represents a single bond, represents oxygen, represents alkanediyl, alkenediyl, oxaalkenediyl or alkinediyl which in each case have up to 4 carbon atoms and which are in each case optionally substituted by halogen, hydroxyl, C₁-C₄-alkyl, C₁-C₄-halogenoalkyl or C₃-C₆-cycloalkyl, or represents one of the following groupings
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- or -N=N-C(R⁴)=N-=-,
where
n represents the numbers 0, 1 or 2,
Q represents oxygen or sulphur,
R⁴ represents hydrogen or cyano, represents alkyl, alkoxy, alkylthio, alkylamino or dialkylamino which in each case have from 1 to 6 carbon atoms in the alkyl groups and which are optionally substituted by halogen, cyano or C₁-C₄-alkoxy, or represents cycloalkyl which has from 3 to 6 carbon atoms and which is in each case optionally substituted by halogen, cyano, carboxyl, C₁-C₄-alkyl or C₁-C₄-alkoxy-carbonyl, and
R⁵ represents hydrogen, hydroxyl or cyano, or represents alkyl which has from 1 to 6 carbon atoms and which is optionally substituted by halogen, cyano or C₁-C₄-alkoxy, or represents cycloalkyl which has from 3 to 6 carbon atoms and which is optionally substituted by halogen, cyano, carboxyl, C₁-C₄-alkyl or C₁-C₄-alkoxy-carbonyl.
R¹ represents hydrogen or straight-chain or branched alkyl having from 1 to 4 carbon atoms.
R² represents hydrogen or straight-chain or branched alkyl having from 1 to 4 carbon atoms.
R³ represents hydrogen or straight-chain or branched alkyl having from 1 to 4 carbon atoms.
X represents oxygen or sulphur.
m represents the numbers 0 or 1, and
Z represents alkyl which has from 1 to 8 carbon atoms and which is optionally substituted by halogen, cyano, hydroxyl, amino, C₁-C₄-alkoxy, C₁-C₄-alkylthio, C₁-C₄-alkylsulphinyl or C₁-C₄-alkylsulphonyl (which are in each case optionally substituted by halogen), represents alkenyl or alkinyl which in each case have up to 8 carbon atoms and which are in each case optionally substituted by halogen, represents cycloalkyl which has from 3 to 6 carbon atoms and which is in each case optionally substituted by halogen, cyano, carboxyl, phenyl (which is optionally substituted by halogen cyano, C₁-C₄-alkyl, C₁-C₄-halogenoalkyl, C₁-C₄-alkoxy or C₁-C₄-halogenoalkoxy), C₁-C₄-alkyl or C₁-C₄-alkoxycarbonyl, or represents in each case optionally substituted phenyl, naphthyl or (optionally benzofuzed) heterocyclyl having 5 or 6 ring members of which at least one represents oxygen, sulphur or nitrogen and, where appropriate, one or two further members represent nitrogen, with the possible substituents preferably being selected from the following list:
oxygen (as a replacement for two geminate hydrogen atoms), halogen, cyano, nitro, amino, hydroxyl, formyl, carboxyl, carbamoyl, thiocarbamoyl, in each case straight-chain or branched alkyl alkoxy alkylthio, alkylsulphinyl or alkylsulphonyl having in each case from 1 to 6 carbon atoms, in each case straight-chain or branched alkenyl or alkenyloxy having in each case from 2 to 6 carbon atoms, in each case straight-chain or branched halogenoalkyl, halogenoalkoxy, halogenoalkylthio, halogenoalkylsulphinyl or halogenoalkylsulphonyl having in each case from 1 to 6 carbon atoms and from 1 to 13 identical or different halogen atoms, in each case straight-chain or branched halogenoalkenyl or halogenoalkenyloxy having in each from 2 to 6 carbon atoms and from 1 to 13 identical or different halogen atoms, in each case straight-chain or branched alkylamino, dialkylamino, alkylcarbonyl, alkylcarbonyloxy, alkoxycarbonyl, alkylsulphonyloxy hydroxyiminoalkyl or alkoxyiminoalkyl having in each case from 1 to 6 carbon atoms in the individual alkyl moieties, in each case doubly linked alkylene or dioxyalkylene which in each case have from 1 to 6 carbon atoms and which are in each case optionally substituted identically or differently, once or more than once, by halogen and/or straight-chain or branched alkyl having from 1 to 4 carbon atoms and/or straight-chain or branched halogenoalkyl having from 1 to 4 carbon atoms and from 1 to 9 identical or different halogen atoms, cycloalkyl having from 3 to 6 carbon atoms, heterocyclyl or heterocyclyl-methyl having in each case from 3 to 7 ring members of which in each case from 1 to 3 are identical or different heteroatoms, in particular nitrogen, oxygen and/or sulphur, and also phenyl, phenoxy, benzyl, benzyloxy, phenylethyl or phenylethyloxy which are in each case optionally substituted identically or differently, once or more than once, in the phenyl moiety by halogen, cyano, nitro, carboxyl, carbamoyl and/or straight-chain or branched alkyl having from 1 to 4 carbon atoms and/or straight-chain or branched halogenoalkyl having from 1 to 4 carbon atoms and from 1 to 9 identical or different halogen atoms and/or straight-chain or branched alkoxy having from 1 to 4 carbon atoms and/or straight-chain or branched halogenoalkoxy having from 1 to 4 carbon atoms and from 1 to 9 identical or different halogen atoms and/or alkylcarbonyl or alkoxycarbonyl having in each case up to 5 carbon atoms.

3. Compounds of the formula (I) according to Claim 1, in which
Ar represents in each case optionally substituted ortho-, -meta- or para- phenylene, represents furandiyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl, pyrimidinediyl, pyridazinediyl, pyrazinediyl, 1,3,4-triazinediyl or 1,2,3-triazinediyl, with the possible substituents being selected, in particular, from the following list:
fluorine, chlorine, cyano, methyl, ethyl, trifluoromethyl, methoxy, ethoxy, methylthio, methylsulphinyl or methylsulphonyl.
G represents a single bond, represents oxygen, represents methylene, dimethylene (ethane-1,2-diyl), ethene-1,2-diyl or ethine-1,2-diyl, which are in each case optionally substituted by fluorine, chlorine, hydroxyl, methyl, ethyl, n- or i-propyl, trifluoromethyl, cyclopropyl, cyclobutyl, cyclopentyl or cyclohexyl, or represents one of the following groupings
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- or -N(R⁵)-CQ-Q-CH₂-,
where
n represents the numbers 0, 1 or 2,
Q represents oxygen or sulphur,
R⁴ represents hydrogen or cyano, or represents methyl, ethyl, n- or i-propyl, n-, ,i-, or s-butyl, methoxy, ethoxy, propoxy, butoxy, methylthio, ethylthio, propylthio, butylthio, methylamino, ethylamino, propylamino, dimethylamino or diethylamino which are optionally substituted by fluorine, chlorine, cyano, methoxy or ethoxy, or represents cyclopropyl, cyclobutyl, cyclopentyl or cyclohexyl which are in each case optionally substituted by fluorine, chlorine, cyano, carboxyl, methyl, ethyl, n- or i-propyl, methoxycarbonyl or ethoxy-carbonyl, and
R⁵ represents hydrogen, hydroxyl or cyano, or represents methyl, ethyl, n- or i-propyl, n-, i-, s- or t-butyl which are in each case optionally substituted by fluorine, chlorine, cyano, methoxy or ethoxy, or represents cyclopropyl, cyclobutyl, cyclopentyl or cyclohexyl which are optionally substituted by fluorine, chlorine, cyano, carboxyl, methyl, ethyl, n- or i-propyl, methoxycarbonyl or ethoxy-carbonyl.
R¹ hydrogen, methyl, ethyl, n- or i-propyl and also n-, i-, s- or t-butyl.
R² represents hydrogen, methyl, ethyl, n- or i-propyl and also n-, i-, s- or t-butyl.
R³ represents hydrogen, methyl, ethyl, n- or i-propyl and also n-, i-, s- or t-butyl.
X represents oxygen or sulphur.
m represents the numbers 0 or 1, and
Z represents methyl, ethyl, n- or i-propyl, n-, i-, s- or t-butyl which are optionally substituted by fluorine, chlorine, bromine, cyano, hydroxyl, amino, methoxy, ethoxy, methylthio, ethylthio, methylsulphinyl, ethylsulphinyl, methylsulphonyl or ethylsulphonyl (which are in each case optionally substituted by fluorine and/or chlorine), represents allyl, crotonyl, 1-methyl-allyl, propargyl or 1-methyl-propargyl which are in each case optionally substituted by fluorine, chlorine or bromine, represents cyclopropyl, cyclobutyl, cyclopentyl or cyclohexyl which are in each case optionally substituted by fluorine, chlorine, bromine, cyano, carboxyl, phenyl (which is optionally substituted by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy), methyl, ethyl, n- or i-propyl, methoxy-carbonyl or ethoxy-carbonyl, or represents in each case optionally substituted phenyl, naphthyl, furyl, tetrahydrofuryl, benzofuryl, tetrahydropyranyl, thienyl, benzothienyl, pyrrolyl, dihydropyrrolyl, tetrahydropyrrolyl, benzopyrrolyl, benzodihydropyrrolyl, oxazolyl, benzoxazolyl, isoxazolyl, thiazolyl, benzthiazolyl, isothiazolyl, imidazolyl, benzimidazolyl, oxadiazolyl, thiadiazolyl, pyridinyl, pyrimidinyl, pyridazinyl, pyrazinyl, 1,2,3-triazinyl, 1,2,4-triazinyl or 1,3,5-triazinyl, with the possible substituents preferably being selected from the following list:
oxygen (as a replacement for two geminate hydrogen atoms), fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl, ethylsulphonyl, trifluoromethyl, difluoromethoxy, trifluoromethoxy, difluoromethylthio, trifluoromethylthio, trifluoromethylsulphinyl, trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroxyiminomethyl, hydroxyiminoethyl methoxyiminomethyl, ethoxyiminomethyl, methoxyiminoethyl or ethoxyiminoethyl; trimethylene (propane-1,3-diyl), methylenedioxy or ethylenedioxy, cyclopropyl, cyclobutyl, cyclopentyl or cyclohexyl which are in each case optionally substituted identically or differently, once or more than once, by fluorine, chlorine, methyl, ethyl, n- or i-propyl, and also phenyl, phenoxy, benzyl or benzyloxy which are in each case optionally substituted identically or differently, once or more than once, in the phenyl moiety by fluorine, chlorine, bromine, cyano, nitro, carboxyl, carbamoyl, methyl, ethyl, n- or i-propyl, n-, i-, s- or t-butyl, trifluoromethyl, methoxy, ethoxy, n- or i-propoxy, difluoromethoxy, trifluoromethoxy, acetyl, methoxycarbonyl or ethoxycarbonyl.

4. Compounds of the formula (I) according to Claim 1, in which
Ar represents ortho-phenylene, pyridine-2,3-diyl or thiophene-2,3-diyl,
G represents oxygen, methylene or one of the following groupings
-CH₂-O-, -O-CH₂-, S(O)ₙ, -CH₂-S(O)ₙ-, S(O)ₙ-CH₂-, -C(R⁴)=N-O-, -O-N=C(R⁴)-, -C(R⁴)=N-O-CH₂-, -N(R⁵)- or -CH₂-O-N=C(R⁴)-,
where
n represents the numbers 0, 1 or 2,
R⁴ represents hydrogen, methyl or ethyl, and
R⁵ represents hydrogen, methyl or ethyl.
R¹ represents methyl.
R² represents hydrogen or methyl.
R³ represents hydrogen or methyl.
X represents oxygen.
m represents the numbers 0 or 1, and
Z represents in each case optionally substituted phenyl, pyridinyl, pyrimidinyl, pyridazinyl, pyrazinyl, 1,2,3-thazinyl, 1,2,4-triazinyl or 1,3,5-triazinyl, with the possible substituents preferably being selected from the following list:
fluorine, chlorine, bromine, cyano, methyl, ethyl, n- or i-propyl, n-, i-, s-or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methoxycarbonyl, ethoxycarbonyl, methoxyiminomethyl, ethoxyiminomethyl, methoxyiminoethyl, ethoxyiminoethyl, methylenedioxy or ethylenedioxy which are in each case optionally substituted identically or differently, once or more than once, by fluorine, chlorine, methyl or ethyl, and also phenyl, phenoxy, benzyl or benzyloxy which are in each case optionally substituted identically or differently, once or more than once, in the phenyl moiety by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy.

5. Process for preparing N-alkoxy-amidine derivatives of the general formula (I), characterized in that N-N-dialkyl-amidine derivatives of the formula (II) 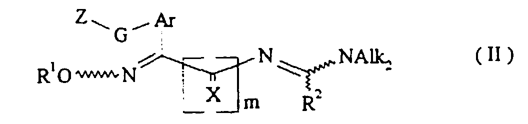 in which
AR, G, R¹, R², X, Z and m have the meaning given in Claim 1, and
Alk represents alkyl,
are reacted with hydroxylamine derivatives of the general formula (III)
H₂N-O-R³ (III)
in which
R³ has the meaning given in Claim 1,
or with their acid addition salts in the presence of a diluent and, where appropriate, in the presence of a reaction auxiliary.

6. N,N-Dialkyl-amidine derivatives of the formula (II) 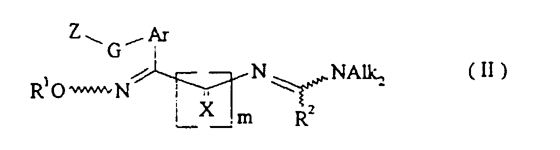 in which
Ar, G, R¹, R², X, Z and m have the meaning given in Claim 1, and
Alk represents alkyl.

7. Pesticides, characterized by a content of at least one compound of the formulae (I) or (II) according to Claims 1 and 6.

8. Process for controlling pests, characterized in that compounds of the formulae (I) or (II) according to Claim 1 are allowed to act on pests and/or their habitat.

9. Use of compounds of the formulae (I) and (II) according to Claims 1 to 6 far controlling pests.

10. Process for preparing pesticides, characterized in that compounds of the formulae (I) or (II) according to Claims 1 to 6 are mixed with extenders and/or surface-active agents.

11. Process for preparing N,N-dialkyl-amidine derivatives of the formula (II) according to Claim 6, characterized in that oxime derivatives of the formula (IV) 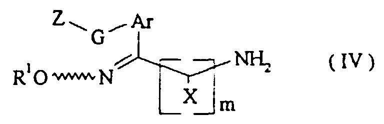 in which
Ar, G, R¹ X, Z and m have the meaning given in Claim 6,
are reacted with aminal esters of the formula (Va) or amide acetals of the formula (Vb) 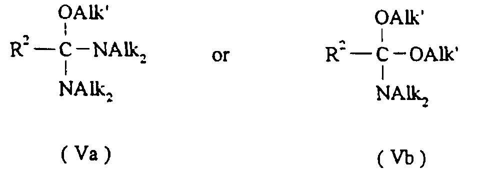 in which
Alk and R² have the meaning given in Claim 6, and
Alk' represents straight-chain or branched alkyl having from 1 to 4 carbon atoms, preferably methyl, ethyl, n- or i-propyl and n-, i-, s- or t-butyl,
where appropriate in the presence of a diluent.

## Revendications

1. Dérivés de N-alkoxy-amidines de formule générale (I) 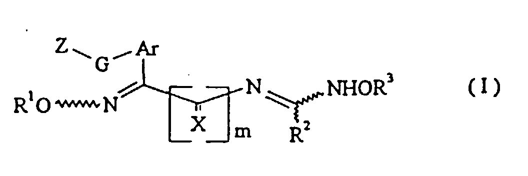 dans laquelle
Ar représente un groupe arylène ou un groupe hétéro-arylène dont chacun est éventuellement substitué ;
G représente une liaison simple, de l'oxygène, un groupe alcanediyle, alcènediyle, oxa-alcènediyle, alcynediyle dont chacun est éventuellement substitué par un radical halogéno, hydroxy, alkyle, halogénalkyle ou cycloalkyle, ou l'un des groupements suivants
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂-ou
-N=N-C(R⁴)=N-O-,
dans lesquels
n représente le nombre 0, 1 ou 2,
Q est l'oxygène ou le soufre,
R¹ est l'hydrogène ou un groupe alkyle ;
R² est l'hydrogène ou un groupe alkyle ;
R³ est l'hydrogène ou un groupe alkyle ;
R⁴ est l'hydrogène, un groupe cyano ou un groupe alkyle, alkoxy, alkylthio, alkylamino, dialkylamino ou cycloalkyle, chacun étant éventuellement substitué, et
R⁵ est l'hydrogène, un groupe hydroxy, cyano, ou bien un groupe alkyle, alkoxy ou cycloalkyle dont chacun est éventuellement substitué ;
X est l'oxygène ou le soufre ;
m représente le nombre 0 ou 1 et
Z est un groupe alkyle, alcényle, alcynyle, cycloalkyle, aryle ou hétérocyclyle, dont chacun est éventuellement substitué.

2. Composés de formule (I) suivant la revendication 1, dans laquelle
Ar est un groupe phénylène ou un groupe naphtylène dont chacun est éventuellement substitué ou un groupe hétéroarylène à cycle de 5 ou 6 atomes, dont au moins l'un est l'oxygène, le soufre ou l'azote et le cas échéant un ou deux autres représentent de l'azote, les substituants possibles étant choisis de préférence dans l'énumération suivante :
halogène, cyano, nitro, amino, hydroxy, formyle, carboxy, carbamoyle, thiocarbamoyle, groupe alkyle, alkoxy, alkylthio, alkylsulfinyle ou alkylsulfonyle ayant chacun 1 à 6 atomes de carbone et étant chacun linéaire ou ramifié, groupe alcényle, alcényloxy ou alcynyloxy ayant chacun 2 à 6 atomes de carbone et étant chacun linéaire ou ramifié, groupe halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et dont chacun est linéaire ou ramifié, groupe halogénalcényle ou halogénalcényloxy ayant chacun 2 à 6 atomes de carbone et 1 à 13 atomes d'halogènes identiques ou différents et dont chacun est linéaire ou ramifié, groupe alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle indidividuelles et étant chacun linéaire ou ramifié, groupe alkylène ou dioxyalkylène étant chacun divalent, ayant chacun 1 à 6 atomes de carbone et portant chacun le cas échéant un ou plusieurs substituants, identiques ou différents, halogéno, et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents, G représente une liaison simple, de l'oxygène, un groupe alcanediyle, alcènediyle, oxa-alcènediyle, alcynediyle ayant chacun jusqu'à 4 atomes de carbone et étant chacun substitué le cas échéant par un radical halogéno, hydroxy, alkyle en C₁ à C₄, halogénalkyle en C₁ à C₄ ou cycloalkyle en C₃ à C₆, ou l'un des groupements suivants :
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- ou -N=N-C(R⁴)=N-=-,
dans lesquels
n représente le nombre 0, 1 ou 2,
Q est l'oxygène ou le soufre,
R⁴ est l'hydrogène, un groupe cyano, un groupe alkyle, alkoxy, alkylthio, alkylamino ou dialkylamino ayant chacun 1 à 6 atomes de carbone dans les parties alkyle et portant éventuellement un substituant halogéno, cyano ou alkoxy en C₁ à C₄, ou un groupe cycloalkyle ayant 3 à 6 atomes de carbone éventuellement substitué par un radical halogéno, cyano, carboxy, alkyle en C₁ à C₄ ou (alkoxy en C₁ à C₄)-carbonyle, et
R⁵ est l'hydrogène, un groupe hydroxy, cyano ou un groupe alkyle ayant 1 à 6 atomes de carbone éventuellement substitué par un radical halogéno, cyano ou alkoxy en C₁ à C₄, ou un groupe cycloalkyle ayant 3 à 6 atomes de carbone éventuellement substitué par un radical halogéno, cyano, carboxy, alkyle en C₁ à C₄ ou (alkoxy en C₁ à C₄)-carbonyle,
R¹ représente l'hydrogène ou un groupe alkyle linéaire ou ramifié ayant 1 à 4 atomes de carbone,
R² représente l'hydrogène ou un groupe alkyle linéaire ou ramifié ayant 1 à 4 atomes de carbone,
R³ représente l'hydrogène ou un groupe alkyle linéaire ou ramifié ayant 1 à 4 atomes de carbone,
X est l'oxygène ou le soufre,
m représente le nombre 0 ou 1 et
Z représente un groupe alkyle de 1 à 8 atomes de carbone éventuellement substitué par des radicaux halogéno, cyano, hydroxy, amino, alkoxy en C₁ à C₄, alkylthio en C₁ à C₄, alkylsulfinyle en C₁ à C₄ ou alkylsulfonyle en C₁ à C₄ (qui sont substitués chacun le cas échéant par un halogène), un groupe alcényle ou alcynyle ayant chacun jusqu'à 8 atomes de carbone et dont chacun est éventuellement substitué par un halogène, un groupe cycloalkyle de 3 à 6 atomes de carbone éventuellement substitué par un radical halogéno, cyano, carboxy, phényle (qui est éventuellement substitué par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle, un groupe phényle, naphtyle ou hétérocyclyle (éventuellement condensé au benzène) à noyau de 5 ou 6 atomes dont l'un au moins est de l'oxygène, du soufre ou de l'azote et, le cas échéant, un ou deux autres représentent de l'azote et chaque groupe étant éventuellement substitué, les substituants possibles étant choisis de préférence dans l'énumération suivante :
oxygène (en remplacement de deux atomes d'hydrogène géminés), halogène, cyano, nitro, amino, hydroxy, formyle, carboxy, carbamoyle, thiocarbamoyle, groupe alkyle, alkoxy, alkylthio, alkylsulfinyle ou alkylsulfonyle ayant chacun 1 à 6 atomes de carbone et étant chacun linéaire ou ramifié, groupe alcényle ou alcényloxy ayant chacun 2 à 6 atomes de carbone et étant chacun linéaire ou ramifié, groupe halogénalkyle, halogénalkoxy, halogénalkylthio, halogénalkylsulfinyle ou halogénalkylsulfonyle ayant chacun 1 à 6 atomes de carbone et 1 à 13 atomes d'halogènes identiques ou différents et dont chacun est linéaire ou ramifié, groupe halogénalcényle ou halogénalcényloxy dont chacun est linéaire ou ramifié et comprend 2 à 6 atomes de carbone et 1 à 13 atomes d'halogènes identiques ou différents, groupe alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et étant chacun linéaire ou ramifié, groupe alkylène ou dioxyalkylène étant chacun divalent et ayant chacun 1 à 6 atomes de carbone, portant chacun le cas échéant un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents, cycloalkyle ayant 3 à 6 atomes de carbone, hétérocyclyle ou hétérocyclylméthyle chacun à noyau de 3 à 7 atomes de carbone dont chaque fois 1 à 3 hétéroatomes sont identiques ou différents - notamment azote, oxygène et/ou soufre -, ainsi que groupe phényle, phénoxy, benzyle, benzyloxy, phényléthyle ou phényléthyloxy dont chacun est éventuellement substitué dans la partie phényle, une ou plusieurs fois identiques ou différentes, par un radical halogéno, cyano, nitro, carboxy, carbamoyle et/ou un radical alkyle linéaire ou ramifié ayant 1 à 4 atomes de carbone et/ou un radical halogénalkyle linéaire ou ramifié ayant 1 à 4 atomes de carbone et 1 à 9 atomes d'halogènes identiques ou différents et/ou un radical alkoxy linéaire ou ramifié ayant 1 à 4 atomes de carbone et/ou un radical halogénalkoxy linéaire ou ramifié ayant 1 à 4 atomes de carbone et 1 à 9 atomes d'halogènes identiques ou différents et/ou un radical alkylcarbonyle ou alkoxycarbonyle ayant chacun jusqu'à 5 atomes de carbone.

3. Composés de formule (I) suivant la revendication 1, dans laquelle
Ar représente un groupe ortho-, méta- ou paraphénylène éventuellement substitué dans chaque cas, un groupe furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle, pyrimidinediyle, pyridazine-diyle, pyrazinediyle, 1,3,4-triazinediyle ou 1,2,3-triazinediyle, les substituants possibles étant choisis en particulier dans l'énumération suivante : fluor, chlore, cyano, méthyle, éthyle, trifluorométhyle, méthoxy, éthoxy, méthylthio, méthylsulfinyle ou méthylsulfonyle,
G est une liaison simple, l'oxygène, un groupe méthylène, diméthylène (éthane-1,2-diyle), éthène-1,2-diyle, éthyne-1,2-diyle dont chacun est éventuellement substitué par du fluor, du chlore, un radical hydroxy, méthyle, éthyle, n-propyle, isopropyle, trifluorométhyle, cyclopropyle, cyclobutyle, cyclopentyle ou cyclohexyle, ou l'un des groupements suivants :
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- ou -N(R⁵)-CQ-Q-CH₂-
dans lesquels
n représente le noire 0, 1 ou 2,
Q est l'oxygène ou le soufre,
R⁴ est l'hydrogène, un groupe cyano, un groupe méthyle, éthyle, n-propyle, isopropyle, n-butyle, isobutyle, sec.-butyle, méthoxy, éthoxy, propoxy, butoxy, méthylthio, éthylthio, propylthio, butylthio, méthylamino, éthylamino, propylamino, diméthylamino ou diéthylamino dont chacun est éventuellement substitué par du fluor, du chlore, un radical cyano, méthoxy ou éthoxy, ou un groupe cyclopropyle, cyclobutyle, cyclopentyle ou cyclohexyle dont chacun est éventuellement substitué par du fluor, du chlore, un radical cyano, carboxy, méthyle, éthyle, n-propyle, isopropyle, méthoxycarbonyle ou éthoxycarbonyle, et
R⁵ représente l'hydrogène, un groupe hydroxy, un groupe cyano, ou un groupe méthyle, éthyle, n-propyle, isopropyle, n-butyle, isobutyle, sec.-butyle, tertio-butyle dont chacun est éventuellement substitué par du fluor, du chlore, un radical cyano, méthoxy ou éthoxy, ou un groupe cyclopropyle, cyclobutyle, cyclopentyle ou cyclohexyle dont chacun est éventuellement substitué par du fluor, du chlore, un radical cyano, carboxy, méthyle, éthyle, n-propyle, isopropyle, méthoxycarbonyle ou éthoxycarbonyle,
R¹ représente l'hydrogène, un groupe méthyle, éthyle, n-propyle, isopropyle, n-butyle, isobutyle, sec.-butyle, ou tertio-butyle,
R² est l'hydrogène, un groupe méthyle, éthyle, n-propyle, isopropyle, n-butyle, isobutyle, sec.-butyle ou tertio-butyle,
R³ est l'hydrogène, un groupe méthyle, éthyle, n-propyle, isopropyle, n-butyle, isobutyle, sec.-butyle ou tertio-butyle,
X est l'oxygène ou le soufre,
m représente le nombre 0 ou 1 et
Z est un groupe méthyle, éthyle, n-propyle, isopropyle, n-butyle, isobutyle, sec.-butyle, tertio-butyle dont chacun est substitué le cas échéant par un radical fluoro, chloro, bromo, cyano, hydroxy, amino, méthoxy, éthoxy, méthylthio, éthylthio, méthylsulfinyle, éthylsulfinyle, méthylsulfonyle, éthylsulfonyle (chacun de ces radicaux étant éventuellement substitué par du fluor et/ou du chlore), un groupe allyle, crotonyle, 1-méthylallyle, propargyle ou 1-méthylpropargyle dont chacun est substitué le cas échéant par du fluor, du chlore ou du brome, un groupe cyclopropyle, cyclobutyle, cyclopentyle ou cyclohexyle dont chacun est substitué le cas échéant par du fluor, du chlore, du brome, un radical cyano, carboxy, phényle (qui est substitué le cas échéant par du fluor, du chlore, du brome, un radical cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy), méthyle, éthyle, n-propyle, isopropyle, méthoxycarbonyle ou éthoxycarbonyle, un groupe phényle, naphtyle, furyle, tétrahydrofuryle, benzofuryle, tétrahydropyrannyle, thiényle, benzothiényle, pyrrolyle, dihydropyrrolyle, tétrahydropyrrolyle, benzopyrrolyle, benzodihydropyrrolyle, oxazolyle, benzoxazolyle, isoxazolyle, thiazolyle, benzothiazolyle, isothiazolyle, imidazolyle, benzimidazolyle, oxadiazolyle, thiadiazolyle, pyridinyle, pyrimidinyle, pyridazinyle, pyrazinyle, 1,2,3-triazinyle, 1,2,4-triazinyle ou 1,3,5-triazinyle dont chacun est éventuellement substitué, les substituants possibles étant choisis de préférence dans l'énumération suivante :
oxygène (en remplacement de deux atomes d'hydrogène géminés), fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle, groupe triméthylène (propane -1,3-diyle), méthylènedioxy, éthylènedioxy, cyclopropyle, cyclobutyle, cyclopentyle ou cyclohexyle dont chacun est substitué le cas échéant une ou plusieurs fois identiques ou différentes par du fluor, du chlore, un radical méthyle, éthyle, n-propyle, isopropyle, ainsi que groupe phényle, phénoxy, benzyle ou benzyloxy portant chacun le cas échéant un ou plusieurs substituants, identiques ou différents, fluoro, chloro, bromo, cyano, nitro, carboxy, carbamoyle, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, trifluorométhoxy, acétyle, méthoxycarbonyle ou éthoxycarbonyle.

4. Composés de formule (I) suivant la revendication 1, dans laquelle
Ar est un groupe ortho-phénylène, pyridine-2,3-diyle ou thiophène-2,3-diyle,
G est l'oxygène, un groupe méthylène ou l'un des groupements suivants
-CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -N(R⁵)- ou -CH₂-O-N=C(R⁴)-
dans lesquels
n a la valeur 0, 1 ou 2,
R⁴ est l'hydrogène, un groupe méthyle ou éthyle et
R⁵ est l'hydrogène, un groupe méthyle ou éthyle,
R¹ est un groupe méthyle,
R² est l'hydrogène ou un groupe méthyle
R³ est l'hydrogène ou un groupe méthyle.
X est l'oxygène.
m a la valeur 0 ou 1 et
Z est un groupe phényle, pyridinyle, pyrimidinyle, pyridazinyle, pyrazinyle, 1,2,3-triazinyle, 1,2,4-triazinyle ou 1,3,5-triazinyle étant chacun éventuellement substitué, les substituants possibles étant choisis de préférence dans l'énumération suivante : fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluoroméhtylsulfinyle, trifluorométhylsulfonyle, méthoxycarbonyle, éthoxycarbonyle, méthoximinométhyle, éthoximinométhyle, méthoximinoéthyle, éthoximinoéthyle, groupe méthylènedioxy ou éthylènedioxy portant chacun le cas échéant un ou plusieurs substituants, identiques ou différents, fluoro, chloro, méthyle ou éthyle, ainsi que groupe phényle, phénoxy, benzyle ou benzyloxy portant chacun le cas échéant dans la partie phényle ou un plusieurs substituants, identiques ou différents, fluoro, chloro, bromo, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy.

5. Procédé de production de dérivés de N-alkoxyamidines de formule générale (I), caractérisé en ce qu'on fait réagir des dérivés de N,N-dialkylamidines de formule (II) 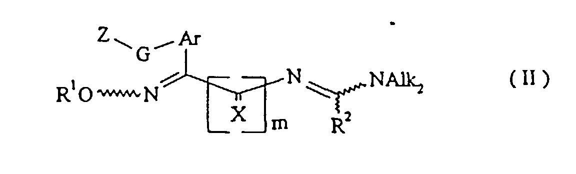 dans laquelle
Ar, G, R¹, R², X, Z et m ont la définition indiquée dans la revendication 1.
et
Alk est un groupe alkyle,
avec des dérivés d'hydroxylamines de formule générale (III)
H₂N - O - R³ (III)
dans laquelle
R³ a la définition indiquée dans la revendication 1,
ou avec leurs sels d'addition d'acides en présence d'un diluant et en la présence éventuelle d'un auxiliaire de réaction.

6. Dérivés de N,N-dialkylamidines de formule (II) 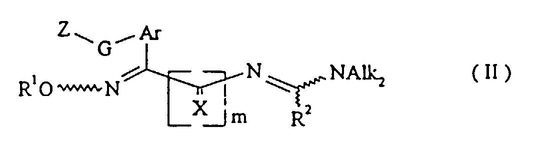 dans laquelle
Ar, G, R¹, R², X, Z et m ont la définition indiquée dans la revendication 1 et
Alk est un groupe alkyle.

7. Compositions pesticides, caractérisées par une teneur en au moins un composé de formules (I) ou (II) suivant les revendications 1 et 6.

8. Procédé pour combattre des parasites, caractérisé en ce qu'on fait agir sur les parasites et/ou sur leur milieu des composés de formules (I) ou (II) suivant la revendication 1.

9. Utilisation de composés de formules (I) et (II) suivant les revendications 1 et 6 pour combattre des parasites.

10. Procédé de préparation de compositions pesticides, caractérisé en ce qu'on mélange des composés de formules (I) ou (II) suivant les revendications 1 à 6 avec des diluants et/ou des agents tensio-actifs.

11. Procédé de production de dérivés de N,N-dialkylamidines de formule (II) suivant la revendication 6, caractérisé en ce qu'on fait réagir des dérivés d'oxime de formule (IV) 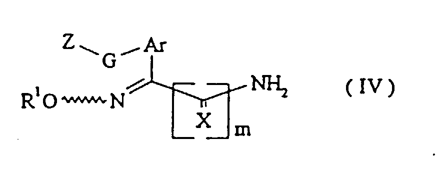 dans laquelle
Ar, G, R¹, X, Z et m ont la définition indiquée dans la revendication 6,
avec des esters d'aminal de formule (Va) ou des amidacétals de formule (Vb) 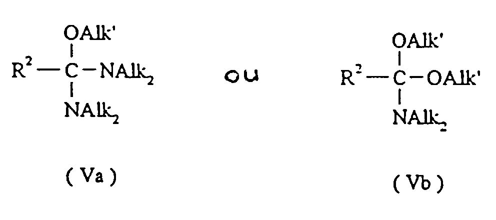 dans lesquelles
Alk et R² ont la définition indiquée dans la revendication 6 et
Alk' est un groupe alkyle linéaire ou ramifié ayant 1 à 4 atomes de carbone, de préférence méthyle, éthyle, n-propyle, isopropyle, n-butyle, isobutyle, sec.-butyle ou tertio-butyle,
le cas échéant en présence d'un diluant.
